# EUROPEAN PATENT APPLICATION

(11) **EP 3 647 435 A1**
(43) Date of publication of application: **06.05.2020**
(21) Application number: 18382771.6
(22) Date of filing: 31.10.2018
(51) Int. Cl.: C12Q 1/6883

(54) **PRECISION PRESCRIPTION OF ENERGY-RESTRICTED PERSONALIZED DIETS WITH DIFFERENT MACRONUTRIENT CONTENT IN SUBJECTS WITH EXCESSIVE BODY WEIGHT**

(71) Applicant: Universidad de Navarra, 31009 Pamplona (Navarra) (ES)
(72) Inventor: Cuervo Zapatel, Marta, 31008 PAMPLONA (ES); Goñi Mateos, Leticia, 31008 PAMPLONA (ES); Martinez Hernandez, José Alfredo, 31008 PAMPLONA (ES); Milagro Yoldi, Fermín Ignacio, 31008 PAMPLONA (ES); Ramos López, Oscar Omar, 31008 PAMPLONA (ES); Riezu Boj, José Ignacio, 31008 PAMPLONA (ES)
(74) Representative: ZBM Patents - Zea, Barlocci & Markvardsen

(57) **Abstract**

The present invention refers to an *in vitro* method to predict the percentage of body mass index (BMI) reduction in response to a diet in a patient with a BMI of at least 25 kg/m² comprising detecting in an isolated sample of the subject the genotype of certain SNPs. The present invention also refers to an *in vitro* method for deciding or recommending a patient with a BMI of at least 25 kg/m² to follow an energy-restricted diet. The present invention also refers to the use of the SNPs of the invention for said methods.

## Description

### Technical Field

The present invention relates to the field of Medicine, and particularly to personalized Medicine in Body Mass Index (BMI) control. It specifically relates to a method and markers for predicting the percentage of BMI reduction in response to two energy-restricted diets.

### Background Art

Overweight and obesity are characterized by abnormal or excessive body fat accumulation that often leads to diverse negative consequences on health. Indeed, a high BMI (Kg/m²) (is a potential risk factor for the onset and development of several chronic diseases, including for example diabetes, cardiovascular events, cancer; accounting for approximately 4 million deaths globally each year.

Energy restriction is one of the most common approaches for obesity treatment, accompanied by prescribing an increase in the level of physical activity (Abete I, 2010). However, not all individuals equally respond to hypocaloric diets, often finding subjects more resistant to reduce the excessive adiposity, or those who rapidly regain the lost weight after leaving the treatment (Martinez JA, 2014). Previous studies have shown that some factors influencing the success concerning weight loss and long-term maintenance in addition to energy restriction include diet composition (distribution of macronutrients, glycemic index, antioxidants intake), or meal frequency, as well as dietary adherence (Bray GA, 2017).

Nevertheless, increasing evidence suggests that interindividual variability in weight loss under a hypocaloric diet also depends upon interactions between genetic and environmental factors, including lifestyle (Qi L, 2014). In this context, nutrigenetic studies have identified a large number of single nucleotide polymorphisms (SNPs) and other structural variants associated with body weight regulation and fuel homeostasis by affecting key biological/physiological processes such as energy expenditure, appetite, adipogenesis, insulin resistance, and lipid metabolism (Goni L, 2016). In this context, no associations were found between a Body Mass Index (BMI)-related Genetic Risk Score (GRS) and changes in body weight over a five-year period of lifestyle counseling in a Danish population (Sandholt CH, 2014). Another study showed that obesity-related GRS influenced the effectiveness of nutritional and lifestyle regimes (based on changes in the dietary intakes of carbohydrates, fat, calorie content and exercise onset) for body fat loss in a large Korean cohort (Cha S, 2018). Interestingly, the mean body fat loss became larger when the genetic sensitivity level to dietary modifications increased (Cha S, 2018). Moreover, highest GRS quartile for diabetes susceptibility was associated with a greater reduction in waist circumference after 1-year of intensive lifestyle intervention (combining dietary fat restriction and increased physical activity) compared to the lowest GRS quartile in patients with obesity and diabetes, although the variance in change attributable to the GRS was relatively small (Peter I, 2012).

There is no current information of how to personally select an appropriate diet among energy-restriction diets in individuals with overweight or obesity. Therefore, there is a need for using precision parameters to optimize individualized prescription of specific nutritional strategies for the prevention and management of excessive body weight.

### Summary of Invention

The inventors have found a personalized method for predicting the percentage of BMI loss dependent on following two energy-restricted diets, a low-fat (LF) diet (20-25% lipids) and a moderately high-protein (MHP) diet (25-35% proteins), in subjects with excessive body weight (subjects with a BMI of at least 25 kg/m²) based on genetic, phenotypical and exogenous factors.

From the data provided below, it is remarkable the fact that using the method of the present invention the a personalized prescription of two energy-restricted diets for percentage of BMI reduction, LF diet and MHP diet, can be performed. Robust statistical approaches were applied to select the best multiple linear regression models explaining the percentage of BMI loss variance in each type of diet. The SNPs (from single nucleotide polymorphisms) found to be associated with the selection of these two energy-restriction diets were the following: rs1801133 (*MTHFR*), rs1052700 (*PLIN1*), rs2605100 (*LYPLAL1*), rs3123554 (*CNR2*), rs10767664 (*BDNF*) and rs659366 (*UCP2*), rs1800544 (*ADRA2A*), rs3813929 (*HTR2C*), rs10182181 (*ADCY3*), rs2943641 (*IRS1*), rs6123837 (*GNAS*), rs662799 (*APOA5*) and rs1042713 (*ADRB2*).

According to the results, predictive values for the percentage of BMI loss variance were found when individually analyzing the effect of each of the mentioned SNPs in the multiple linear regression models (see tables 5 and 6). Unweighted and weighted Genetic Risk Scores (uGRS and wGRS, respectively) were developed using those SNPs for each diet with surprising results for both cases in the prediction of % of BMI loss in subjects with excessive body weight (see tables 5 and 6). Percentage of BMI loss in the MHP was predicted about 28% by a weighted genetic risk score (wGRS) comprising the detection of the SNPs rs1801133 (*MTHFR*), rs1052700 (*PLIN1*), rs2605100 (*LYPLAL1*), rs3123554 (*CNR2*), rs10767664 (*BDNF*) and rs659366 (*UCP2*), in combination with age. On the other hand, another wGRS comprising the detection of the SNPS rs1800544 (*ADRA2A*), rs3813929 (*HTR2C*), rs10182181 (*ADCY3*), rs2943641 (*IRS1*), rs6123837 (*GNAS*), rs662799 (*APOA5*) and rs1042713 (*ADRB2*) and the combination with baseline energy intake explained approximately 29% of percentage of BMI loss variability in the LF diet. The incorporation of these predictive models into a decision algorithm allowed prescribing a specific diet (MHP or LF) or taking into account individual food preferences to precisely choose between those two diets.

The first aspect of the invention refers to an *in vitro* method to predict the percentage of BMI reduction in response to a diet in a patient with a BMI of at least 25 kg/m² comprising detecting in an isolated sample of the subject the genotype of one or more single nucleotide polymorphisms (SNPs) selected from the following list: rs1801133 (*MTHFR*), rs1052700 (*PLIN1*), rs2605100 (*LYPLAL1*), rs3123554 (*CNR2*), rs10767664 (*BDNF*), rs659366 (*UCP2*) and combinations thereof; and the detection of the genotype of one or more SNPs selected from the following list: rs1800544 (*ADRA2A*), rs3813929 (*HTR2C*), rs10182181 (*ADCY3*), rs2943641 (*IRS1*), rs6123837 (*GNAS*), rs662799 (*APOA5*), rs1042713 (*ADRB2*) and combinations thereof; wherein the diet is an energy-restricted diet selected from a diet wherein the total energy (E) intake from lipids is 20-25% (LF diet); or, alternatively, a diet wherein the E from proteins is 25-35% (MHP diet).

The second aspect of the invention refers to an *in vitro* method for deciding or recommending a patient with a BMI of at least 25 kg/m² to follow an energy-restricted diet for reduction of the percentage of BMI, selected from a LF diet wherein the total E intake from lipids is 20-25%; or a MHP diet wherein the E from proteins is 25-35%; wherein the method comprises detecting in an isolated sample of the subject the genotype of one or more SNPs selected from the following list: rs1801133 (*MTHFR*), rs1052700 (*PLIN1*), rs2605100 (*LYPLAL1*), rs3123554 (*CNR2*), rs10767664 (*BDNF*)*,* rs659366 (*UCP2*) and combinations thereof; and the detection of at least one or more SNPs selected form the following list: rs1800544 (*ADRA2A*), rs3813929 (*HTR2C*), rs10182181 (*ADCY3*), rs2943641 (*IRS1*), rs6123837 (*GNAS*), rs662799 (*APOA5*), rs1042713 (*ADRB2*) and combinations thereof.

The third aspect of the invention refers to the use of means for detecting the genotypes as defined in the first or the second aspect of the invention in a biological sample from a subject for the *in vitro* prediction of the reduction of the percentage of BMI in response to a diet of the first aspect of the invention or the *in vitro* method for decision or recommendation of a patient to follow an energy-restricted diet as defined of the second aspect of the invention.

The fourth aspect of the invention refers to the use of at least one of the following SNPs for the *in vitro* prediction of the reduction of the percentage of BMI in response to a diet as defined in the first aspect of the invention or for the *in vitro* method for decision or recommendation of a patient to follow an energy-restricted diet as defined in the second aspect of the invention, which is selected from the following list: rs1801133 (*MTHFR*), rs1052700 (*PLIN1*), rs2605100 (*LYPLAL1*), rs3123554 (*CNR2*), rs10767664 (*BDNF*), rs659366 (*UCP2*), rs1800544 (*ADRA2A*), rs3813929 (*HTR2C*), rs10182181 (*ADCY3*), rs2943641 (*IRS1*), rs6123837 (*GNAS*), rs662799 (*APOA5*), rs1042713 (*ADRB2*) and combinations thereof.

The fifth aspect of the invention refers to the use of at least one of the following genotypes for the *in vitro* prediction of the reduction of the percentage of BMI in response to a diet as defined in the first aspect of the invention or for the *in vitro* method for decision or recommendation of a patient to follow an energy-restricted diet as defined in the second aspect of the invention, which is selected from the following list: AA of rs1801133, TT of rs1052700, GG of rs2605100, AA or AG of rs3123554, TA of rs10767664, CC of rs659366, GG or GC of rs1800544, CC or TT of rs3813929, AG or GG of rs10182181, TC or CC of rs2943641, GA of rs6123837, GG or AA of rs662799, GA of rs1042713 and combinations thereof.

The sixth aspect of the invention refers to a method of treatment of a subject with a BMI of at least 25 kg/m² with an energy-restricted diet selected from a diet wherein the total energy (E) intake from lipids is 20-25% (LF diet); or, alternatively, a diet wherein the E from proteins is 25-35%, comprising detecting in an isolated sample of the subject the genotype of one or more (SNPs selected from the following list: rs1801133 (*MTHFR*), rs1052700 (*PLIN1*), rs2605100 (*LYPLAL1*), rs3123554 (*CNR2*), rs10767664 (*BDNF*), rs659366 (*UCP2*) and combinations thereof; and the detection of the genotype of one or more SNPs selected from the following list: rs1800544 (*ADRA2A*), rs3813929 (*HTR2C*), rs10182181 (*ADCY3*), rs2943641 (*IRS1*), rs6123837 (*GNAS*), rs662799 (*APOA5*), rs1042713 (*ADRB2*) and combinations thereof; wherein when the subject is considered at risk of non-% BMI loss using LF diet, then is treated using MHP diet, whereas, when the subject is considered at risk of non-% BMI loss using MHP diet, then is treated using LF diet.

### Brief Description of Drawings

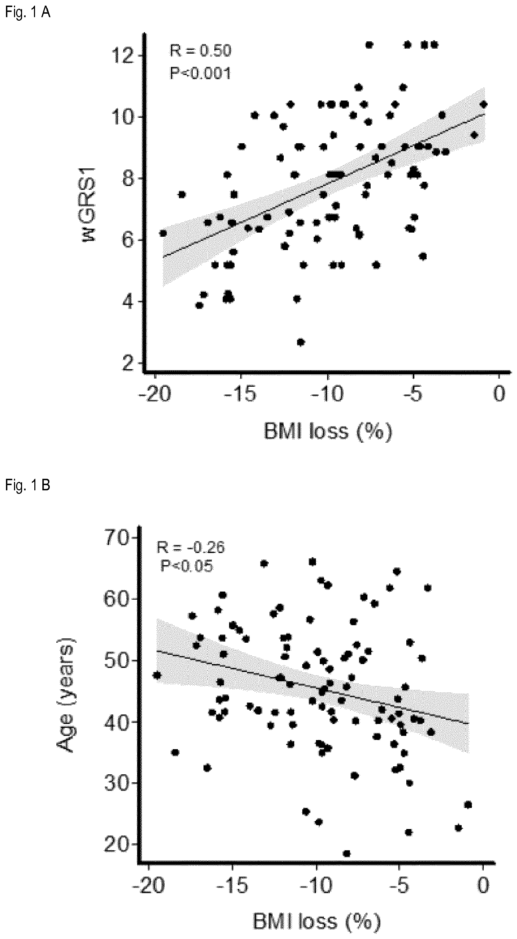

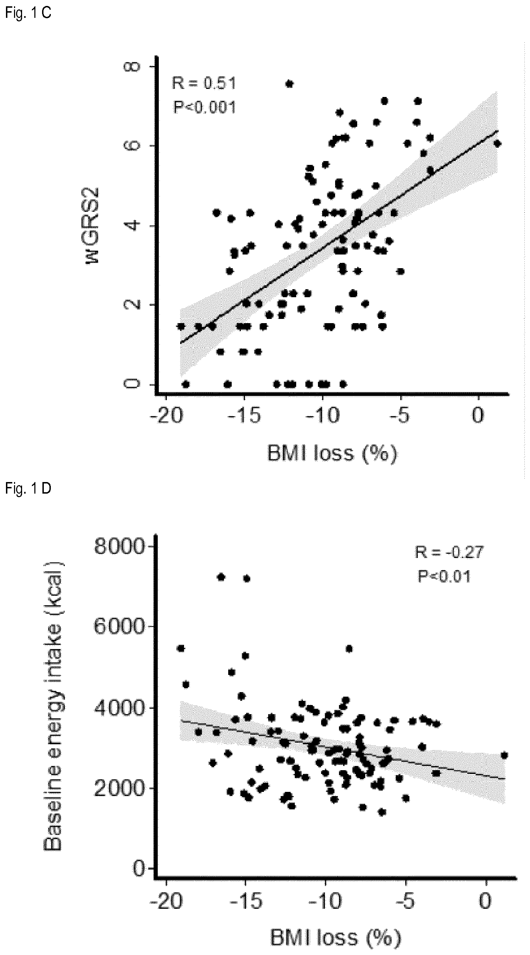

Fig. 1 shows correlations of statistically significant genetic, phenotypical and environmental factors predicting BMI loss depending on dietary prescription. P values were obtained from Pearson correlation tests. Fig. 1A and 1B correspond to MHP diet. Figs. 1C and 1D correspond to LF diet. MHP: moderately high-protein diet; LF: low-fat diet; wGRS1: genetic risk score for MHP diet; wGRS2: genetic risk score for LF diet.

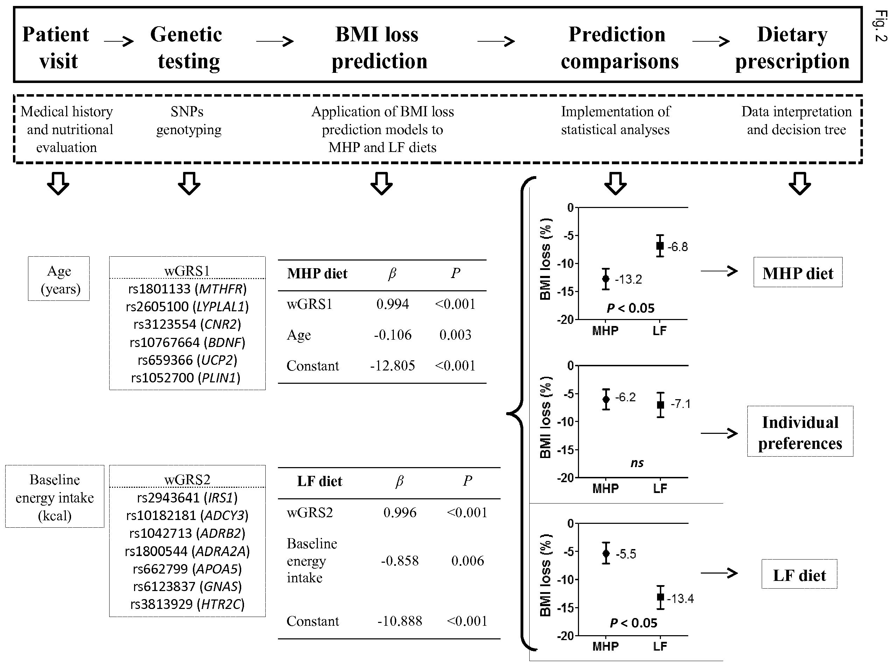

Fig. 2 shows the decision algorithm for personalized dietary treatment of BMI loss based on genetic, phenotypical, and environmental data. wGRS1: genetic risk score for MHP diet; wGRS2: genetic risk score for LF diet.

### Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

The first aspect of the invention refers to an *in vitro* method to predict the percentage of BMI reduction in response to a diet in a patient with a BMI of at least 25 kg/m² comprising detecting in an isolated sample of the subject the genotype of one or more SNPs selected from the following list: rs1801133 (*MTHFR*), rs1052700 (*PLIN1*), rs2605100 (*LYPLAL1*), rs3123554 (*CNR2*), rs10767664 (*BDNF*), rs659366 (*UCP2*) and combinations thereof; and the detection of the genotype of one or more SNPs selected from the following list: rs1800544 (*ADRA2A*), rs3813929 (*HTR2C*), rs10182181 (*ADCY3*), rs2943641 (*IRS1*), rs6123837 (*GNAS*), rs662799 (*APOA5*), rs1042713 (*ADRB2*) and combinations thereof; wherein the diet is an energy-restricted diet selected from a diet wherein the total energy (E) intake from lipids is 20-25% (LF diet); or, alternatively, a diet wherein the E from proteins is 25-35% (MHP diet).

In the present invention "subjects with excessive body weight" refers to subjects with overweight and also to subjects with obesity. A subject with overweight is considered to be a person with a BMI ≥ 25 kg/m² and <30 kg/m². A subject with obesity is considered to a person with a BMI of at least 30 kg/m². Therefore, the present invention is useful for subjects with a BMI of at least 25 kg/m².

BMI is calculated, by the methods known by the skilled person, as the ratio between kilograms by squared meters (kg/m²),derived from the mass (weight, in kilograms) and height (in meters) of an individual.

In the present invention the terms "percentage of BMI loss", "percentage of BMI reduction", "% of BMI loss", "% of BMI reduction", BMI loss (%), BMI reduction (%) have the same meaning, that is, the reduction of the percentage of BMI in a subject after the following of the energy-restricted diets described in the present invention, that is, the reduction (measured in percentage) of BMI or the reduction in BMI in percent.

In an embodiment of the first aspect of the invention, the diet is an energy-restricted diet selected from a diet wherein the total energy (E) intake from carbohydrates is 55-65%, E from proteins is 15-20% and E from lipids is 20-25% (LF diet); or, alternatively, the diet is a diet wherein the E from carbohydrates is 40-50%, E from proteins is 25-35% and E from lipids is 28-35% (MHP diet).

In an embodiment of the first aspect of the invention, the diet is an energy-restricted diet selected from a diet wherein the total energy (E) intake from carbohydrates is 60%, E from proteins is 18% and E from lipids is 22% (LF diet); or, alternatively, the diet is a diet wherein the E from carbohydrates is 40%, E from proteins is 30% and E from lipids is 30% (MHP diet).

In an embodiment of any of the aspects of the present invention, the energy content of LF and MHP diets is at least 1200 kcal/day.

In an embodiment of any of the aspects of the present invention, the subject does not follow physical activity increase.

In an embodiment of any of the aspects of the present invention, the subject of the present invention is Caucasian.

In the sense of the invention, the expression "detecting in an isolated sample of the subject the genotype" encompasses detecting, confirming presence or absence of a particular genotype which is correlated with the risk or no-risk of BMI percentage loss following a particular energy-restriction diet.

Any genotyping technique may be used to detect the genotypes defined in the present invention. In one embodiment, the determination of the genotype is carried out by one of the techniques selected from the group consisting of primer-specific PCR followed by detection or sequencing, primer-specific PCR multiplex followed by detection or sequencing, multiplex allele specific primer extension, an DNA hybridization microarray-based method, dynamic allele-specific hybridization, and DNA fragmentation followed by amplification and sequencing. In a particular embodiment, it is carried out by primer-specific PCR followed by detection.

The polymerase chain reaction (PCR) is the most widely used method for the *in vitro* amplification of nucleic acids. The PCR can be a real-time PCR, wherein the detection by labelled probes of the presence of the target genotypes is almost instantaneous to the amplification.

The amplification of the target polymorphisms can be performed by primer-specific PCR multiplex with following detection by polyacrylamide electrophoresis or by analysis with a genetic analyzer. Alternatively, various PCR reactions can be performed followed by agarose gel electrophoresis or by sequencing.

Determination of the genotype can be performed by Allele Specific Primer Extension (ASPE). This is a sequence specific enzymatic reaction technology that can be used to assay multiple SNPs in a single tube. The ASPE method involves two phases, an enzymatic reaction that determines the target genotype followed by a capture on solid microsphere surface for detection. Taking advantage of the solution phase kinetics, this technique allows sequence labelled microspheres to be used for detecting new templates. This procedure is done with the help of an appropriate capture sequence attached to the allele specific oligonucleotide.

Optionally, detection may be carried out by DNA biochips/microarrays made with oligonucleotides deposited by any mechanism, with oligonucleotides synthesized in situ by photolithography or any other mechanism. A microarray-based method that allow multiplex SNP genotyping in total human genomic DNA without the need for target amplification or complexity reduction can also be used for the genotyping of the variations. This direct SNP genotyping methodology requires no enzymes and relies on the high sensitivity of the gold nanoparticle probes. Specificity is derived from two sequential oligonucleotide hybridizations to the target by allele-specific surface-immobilized capture probes and gene-specific oligonucleotide-functionalized gold nanoparticle probes. The assay format is simple, rapid and robust pointing to its suitability for multiplex SNP profiling at the 'point of care'.

Furthermore, determination of the genotype can be performed by dynamic allele-specific hybridization (DASH), which represents the basis for throughput SNP genotyping in some laboratories. The core reaction principal of DASH is real-time (dynamic) tracking of allele-specific differences in the process of DNA denaturation. To achieve this, an oligonucleotide probe is first hybridized to the target DNA, a necessary component of essentially all genotyping methods. The target DNA comprises one strand of a PCR product immobilized onto a solid surface, and a single probe is used that is complementary to one of the target alleles. This assay concept was shown to be very precise (>99.9% accurate).

In an embodiment of the present invention, genotyping is performed by targeted next generation sequencing. In another embodiment, the targeted next generation sequencing is performed in an Ion Torrent PGM™ equipment.

The term "single-nucleotide polymorphism", often abbreviated to SNP, and also termed in this description "genetic variant", is a variation in a single nucleotide that occurs at a specific position in the genome, where each variation is present to some appreciable degree within a population (e.g. > 1%). In some occasions, prior art also refers with SNP few nucleotide changes, as well as small deletions or insertions (indels) if they are with a frequency of > 1% in a population. SNPs references in this description correspond with accession numbers of dbSNP public database, according to assembly GRCh37.p13. The two alleles of the SNP are indicated with the following notation: NN of rs rsnnnnnn, wherein N is any of the nucleotides A, T, C, G (base) and n is a numeric digit in the identification of SNP in the database (e.g. AA of rs1801133 means that the two alleles in the genome of the identification rs are A and A, or that the genotype is defined by two As). The "genotype of a SNP" is to be understood as the nucleotide (base) present in each of the alleles in the subject.

In the present invention the Hardy-Weinberg equilibrium (HWE) for all genetic variants can estimated using, for example, the Convert program (Version 1.31) and the Arlequin software (Version 3.0).

The following SNPs are the SNPs of the present invention (release date of 30^{th} October 2018):
rs1801133 is located in exon region of *Methylenetetrahydrofolate Reductase* (MTHFR) gene, in human chromosome 1 position 11856378 (according to assembly GRCh37.p13), and its alleles are G/A.
rs1052700 is located in UTR-3 region of *Perilipin 1* (PLIN1) gene, in human chromosome 15 position 90208310 (according to assembly GRCh37.p13) and its alleles are A/T.
rs2605100 is located near the *lysophospholipase like 1* (LYPLAL1) gene, in human chromosome 1 position 219644224 (according to assembly GRCh37.p13), and its alleles are A/G.
rs3123554 is located near the *cannabinoid receptor 2* (CNR2) gene, in human chromosome 1 position 24196401 (according to assembly GRCh37.p13), and its alleles are A/G.
rs10767664 is located in exon region of brain derived *neurotrophic factor* (BDNF) gene, in human chromosome 11 position 27725986 (according to assembly GRCh37.p13), and its alleles are T/A.
rs659366 is located near the *uncoupling protein* 2 (UCP2) gene, in human chromosome 11 position 73694754 (according to assembly GRCh37.p13), and its alleles are C/T.
rs1800544 is located near the *adrenoceptor alpha 2A* (ADRA2A) gene, in human chromosome 10 position 112836503 (according to assembly GRCh37.p13), and its alleles are G/C.
rs3813929 is located near the *5-hydroxytryptamine receptor 2C* (HTR2C) gene, in human chromosome X position 113818520 (according to assembly GRCh37.p13), and its alleles are C/T.
rs10182181 is located near the *adenylate cyclase* 3 (ADCY3) gene, in human chromosome 2 position 25150296 (according to assembly GRCh37.p13), and its alleles are A/G.
rs2943641 is located near the *insulin receptor substrate 1* (IRS1) gene, in human chromosome 2 position 227093745 (according to assembly GRCh37.p13), and its alleles are T/C.
rs6123837 is located in intron region of *GNAS complex locus* (GNAS) gene, in human chromosome 20 position 57465571 (according to assembly GRCh37.p13), and its alleles are G/A.
rs662799 is located near the *apolipoprotein A5* (APOA5) gene, in human chromosome 11 position 116663707 (according to assembly GRCh37.p13), and its alleles are G/A.
rs1042713 is located in exon region of *adrenoceptor beta 2* (ADRB2) gene, in human chromosome 5 position 148206440 (according to assembly GRCh37.p13), and its alleles are G/A.

Energy-restricted diet as referred herein is the diet that accounts for a 30% restriction of the total energy expenditure (for example, as explained in Lopez-Legarrea P, 2014).

The diet that provides 60% of total energy (E) intake from carbohydrates, 18% E from proteins, and 22% E from lipids, is a low fat (LF) (LF diet or diet L) as described in the Nutrient-Gene Interactions in Human Obesity trial (Handjieva-Darlenska T, 2012).

The diet that supplies 40% E from carbohydrates, 30% E from proteins, and 30% E from lipids is a moderately high-protein (MHP) (MHP diet or diet M) based on the Metabolic Syndrome Reduction in Navarra, Spain, study criteria (de la Iglesia R, 2014).

In an embodiment of the first aspect of the invention, the *in vitro* method to predict the % of BMI reduction in response to a diet comprises detecting the genotype of one, two, three, four, five or six of the SNPs rs1801133, rs1052700, rs2605100, rs3123554, rs10767664 and rs659366; and the detection of the genotype of one, two, three, four, five, six or seven of the SNPs rs1800544, rs3813929, rs10182181, rs2943641, rs6123837, rs662799 and rs1042713; or any combinations thereof.

In an embodiment of the first aspect of the invention, the *in vitro* method to predict the % of BMI reduction in response to a diet comprises detecting the genotype of rs1801133, and the detection of the genotype of one, two, three, four, five, six or seven of the SNPs rs1800544, rs3813929, rs10182181, rs2943641, rs6123837, rs662799 and rs1042713; or alternatively, detecting the genotype of rs1801133 and rs1052700, and the detection of the genotype of one, two, three, four, five, six or seven of the SNPs rs1800544, rs3813929, rs10182181, rs2943641, rs6123837, rs662799 and rs1042713; or alternatively, detecting the genotype of rs1801133, rs1052700 and rs2605100, and the detection of the genotype of one, two, three, four, five, six or seven of the SNPs rs1800544, rs3813929, rs10182181, rs2943641, rs6123837, rs662799 and rs1042713; or alternatively, detecting the genotype of rs1801133, rs1052700, rs2605100 and rs3123554, and the detection of the genotype of one, two, three, four, five, six or seven of the SNPs rs1800544, rs3813929, rs10182181, rs2943641, rs6123837, rs662799 and rs1042713; or alternatively, detecting the genotype of rs1801133, rs1052700, rs2605100, rs3123554 and rs10767664, and the detection of the genotype of one, two, three, four, five, six or seven of the SNPs rs1800544, rs3813929, rs10182181, rs2943641, rs6123837, rs662799 and rs1042713; or alternatively, detecting the genotype of rs1801133, rs1052700, rs2605100, rs3123554, rs10767664 and rs659366, and the detection of the genotype of one, two, three, four, five, six or seven of the SNPs rs1800544, rs3813929, rs10182181, rs2943641, rs6123837, rs662799 and rs1042713.

In an embodiment of the first aspect of the invention, the *in vitro* method to predict the % of BMI reduction in response to a diet comprises detecting the genotype of one, two, three, four, five or six of the SNPs rs1801133, rs1052700, rs2605100, rs3123554, rs10767664 and rs659366, and the detection of the genotype of rs1800544; or alternatively, comprises detecting the genotype of one, two, three, four, five or six of the SNPs rs1801133, rs1052700, rs2605100, rs3123554, rs10767664 and rs659366, and the detection of the genotype of rs1800544 and rs3813929; or alternatively, comprises detecting the genotype of one, two, three, four, five or six of the SNPs rs1801133, rs1052700, rs2605100, rs3123554, rs10767664 and rs659366, and the detection of the genotype of rs1800544, rs3813929 and rs10182181; or alternatively, comprises detecting the genotype of one, two, three, four, five or six of the SNPs rs1801133, rs1052700, rs2605100, rs3123554, rs10767664 and rs659366, and the detection of the genotype of rs1800544, rs3813929, rs10182181 and rs2943641; or alternatively, comprises detecting the genotype of one, two, three, four, five or six of the SNPs rs1801133, rs1052700, rs2605100, rs3123554, rs10767664 and rs659366, and the detection of the genotype of rs1800544, rs3813929, rs10182181, rs2943641 and rs6123837; or alternatively, comprises detecting the genotype of one, two, three, four, five or six of the SNPs rs1801133, rs1052700, rs2605100, rs3123554, rs10767664 and rs659366, and the detection of the genotype of rs1800544, rs3813929, rs10182181, rs2943641, rs6123837 and rs662799; or alternatively, comprises detecting the genotype of one, two, three, four, five or six of the SNPs rs1801133, rs1052700, rs2605100, rs3123554, rs10767664 and rs659366, and the detection of the genotype of rs1800544, rs3813929, rs10182181, rs2943641, rs6123837, rs662799 and rs1042713.

In another embodiment of the first aspect of the invention, the *in vitro* method comprises detecting the genotype of the SNPs rs1801133, rs1052700, rs2605100, rs3123554, rs10767664 and rs659366; and the detection of the genotype of the SNPs rs1800544, rs3813929, rs10182181, rs2943641, rs6123837, rs662799 and rs1042713.

In another embodiment of the first aspect of the invention, when one or more of the genotypes selected from the following list is detected, it is indicative of reduction of the % of BMI of MHP diet: AA of rs1801133, TT of rs1052700, GG of rs2605100, AA or AG of rs3123554, TA of rs10767664, CC of rs659366 and combinations thereof; or alternatively, when one or more of the genotypes selected from the following list is detected, it is indicative of reduction of the % of BMI of the LF diet: GG or GC of rs1800544, CC or TT of rs3813929, AG or GG of rs10182181, TC or CC of rs2943641, GA of rs6123837, GG or AA of rs662799, GA of rs1042713 and combinations thereof.

In another embodiment of the first aspect of the invention, when one, two, three, four, five or six of the genotypes selected from the following list is detected, it is indicative of reduction of the % of BMI of MHP diet: AA of rs1801133, TT of rs1052700, GG of rs2605100, AA or AG of rs3123554, TA of rs10767664, CC of rs659366 and combinations thereof; or alternatively, when one, two, three, four, five, six or seven of the genotypes selected from the following list is detected, it is indicative of reduction of the % of BMI of the LF diet: GG or GC of rs1800544, CC or TT of rs3813929, AG or GG of rs10182181, TC or CC of rs2943641, GA of rs6123837, GG or AA of rs662799, GA of rs1042713 and combinations thereof.

In another embodiment of the first aspect of the invention, when the genotype AA of rs1801133 is detected; or alternatively, when AA of rs1801133 and TT of rs1052700 are detected; or alternatively, when AA of rs1801133, TT of rs1052700 and GG of rs2605100 are detected; or alternatively, when AA of rs1801133, TT of rs1052700, GG of rs2605100 and AA or AG of rs3123554 are detected; or alternatively, when AA of rs1801133, TT of rs1052700, GG of rs2605100, AA or AG of rs3123554 and TA of rs10767664 are detected; or alternatively when AA of rs1801133, TT of rs1052700, GG of rs2605100, AA or AG of rs3123554, TA of rs10767664 and CC of rs659366 are detected; it is indicative of reduction of the % of BMI of MHP diet.

In another embodiment of the first aspect of the invention, when the genotype GG or GC of rs1800544 is detected; or alternatively, when GG or GC of rs1800544 and CC or TT of rs3813929 are detected; or alternatively, when GG or GC of rs1800544, CC or TT of rs3813929 and AG or GG of rs10182181 are detected; or alternatively, when GG or GC of rs1800544, CC or TT of rs3813929, AG or GG of rs10182181 and TC or CC of rs2943641 are detected; or alternatively, when GG or GC of rs1800544, CC or TT of rs3813929, AG or GG of rs10182181, TC or CC of rs2943641 and GA of rs6123837 are detected; or alternatively, when GG or GC of rs1800544, CC or TT of rs3813929, AG or GG of rs10182181, TC or CC of rs2943641, GA of rs6123837 and GG or AA of rs662799; or alternatively, when GG or GC of rs1800544, CC or TT of rs3813929, AG or GG of rs10182181, TC or CC of rs2943641, GA of rs6123837, GG or AA of rs662799 and GA of rs1042713 are detected; it is indicative of reduction of the % of BMI of the LF diet.

In another embodiment of the first aspect of the invention, when the following genotypes are detected it is indicative of reduction of the % of BMI of the MHP diet: AA of rs1801133, TT of rs1052700, GG of rs2605100, AA or AG of rs3123554, TA of rs10767664 and CC of rs659366; and, when the following genotypes are detected, it is indicative of reduction of the % of BMI of the LF diet: GG or GC of rs1800544, CC or TT of rs3813929, AG or GG of rs10182181, TC or CC of rs2943641, GA of rs6123837, GG or AA of rs662799 and GA of rs1042713.

The present invention also refers to the detection of the genotypes of the above mentioned SNPs which are indicative of non- reduction of the % of BMI of MHP diet and/or LF diet.

Therefore, in another embodiment of the first aspect of the invention, when one or more of the genotypes selected from the following list is detected, it is indicative of non-reduction of the % of BMI of MHP diet: GG or GA of rs1801133, AA or AT of rs1052700, AA or AG of rs2605100, GG of rs3123554, TT or AA of rs10767664, CT or TT of rs659366 and combinations thereof; or alternatively, when one or more of the genotypes selected from the following list is detected, it is indicative of non-reduction of the % of BMI of the LF diet: CC of rs1800544, CT of rs3813929, AA of rs10182181, TT of rs2943641, GG or AA of rs6123837, GA of rs662799, GG or AA of rs1042713 and combinations thereof.

In another embodiment of the first aspect of the invention, when the genotype GG or GA of rs1801133 is detected; or alternatively, when GG or GA of rs1801133 and AA or AT of rs1052700 are detected; or alternatively, when GG or GA of rs1801133, AA or AT of rs1052700 and AA or AG of rs2605100 are detected; or alternatively, when GG or GA of rs1801133, AA or AT of rs1052700, AA or AG of rs2605100 and GG of rs3123554 are detected; or alternatively, when GG or GA of rs1801133, TT of rs1052700, AA or AG of rs2605100, GG of rs3123554 and TT or AA of rs10767664 are detected; or alternatively, when GG or GA of rs1801133, AA or AT of rs1052700, AA or AG of rs2605100, GG of rs3123554, TT or AA of rs10767664 and CT or TT of rs659366 are detected; it is indicative of non-reduction of the % of BMI of MHP diet.

In another embodiment of the first aspect of the invention, when CC of rs1800544 is detected; or alternatively, when CC of rs1800544 and CT of rs3813929 are detected; or alternatively, when CC of rs1800544, CT of rs3813929 and AA of rs10182181 are detected; or alternatively, when CC of rs1800544, CT of rs3813929, AA of rs10182181 and TT of rs2943641 are detected; or alternatively, when CC of rs1800544, CT of rs3813929, AA of rs10182181, TT of rs2943641 and GG or AA of rs6123837 are detected; or alternatively, when CC of rs1800544, CT of rs3813929, AA of rs10182181, TT of rs2943641, GG or AA of rs6123837 and GA of rs662799 are detected; or alternatively, when CC of rs1800544, CT of rs3813929, AA of rs10182181, TT of rs2943641, GG or AA of rs6123837, GA of rs662799 and GG or AA of rs1042713 are detected, it is indicative of non-reduction of the % of BMI of LF diet.

In another embodiment of the first aspect of the invention, when GG or GA of rs1801133, AA or AT of rs1052700, AA or AG of rs2605100, GG of rs3123554, TT or AA of rs10767664 and CT or TT of rs659366 are detected; it is indicative of non-reduction of % of BMI of MHP diet; and/or when CC of rs1800544, CT of rs3813929, AA of rs10182181, TT of rs2943641, GG or AA of rs6123837, GA of rs662799 and GG or AA of rs1042713 are detected, it is indicative of non-reduction of the % of BMI of LF diet.

The second aspect of the invention refers to an *in vitro* method for deciding or recommending a patient with a BMI of at least 25 kg/m² to follow an energy-restricted diet, for reduction of percentage of BMI, selected from a LF diet wherein the total E intake from lipids is 20-25%; or a MHP diet wherein the E from proteins is 25-35% wherein the method comprises detecting in an isolated sample of the subject the genotype of one or more SNPs selected from the following list: rs1801133, rs1052700, rs2605100, rs3123554, rs10767664, rs659366 and combinations thereof; and the detection of the genotype of at least one or more SNPs selected form the following list: rs1800544, rs3813929, rs10182181, rs2943641, rs6123837, rs662799, rs1042713 and combinations thereof.

In an embodiment of the second aspect of the invention, the method the energy-restricted diet for reduction of percentage of BMI, selected from a LF diet wherein the total E intake from carbohydrates is 55-65%, E from proteins is 15-20% and E from lipids is 20-25%; or a MHP diet wherein the E from carbohydrates is 40-50%, E from proteins is 25-35% and E from lipids is 28-35%.

In an embodiment of the second aspect of the invention, the method the energy-restricted diet for reduction of percentage of BMI, selected from a LF diet wherein the total E intake from carbohydrates is 60%, E from proteins is 18% and E from lipids is 22%; or a MHP diet wherein the E from carbohydrates is 40%, E from proteins is 30% and E from lipids is 30%.

In an embodiment of the second aspect of the invention, the method comprises detecting in an isolated sample of the subject the genotype of one, two, three, four, five or six SNPs selected from the following list: rs1801133, rs1052700, rs2605100, rs3123554, rs10767664, rs659366 and combinations thereof; and the detection of the genotype of one, two, three, four, five, six or seven SNPs selected form the following list: rs1800544, rs3813929, rs10182181, rs2943641, rs6123837, rs662799, rs1042713 and combinations thereof.

In an embodiment of the second aspect of the invention, the method comprises detecting the genotype of rs1801133, and the detection of the genotype of one, two, three, four, five, six or seven of the SNPs rs1800544, rs3813929, rs10182181, rs2943641, rs6123837, rs662799 and rs1042713; or alternatively, detecting the genotype of rs1801133 and rs1052700, and the detection of the genotype of one, two, three, four, five, six or seven of the SNPs rs1800544, rs3813929, rs10182181, rs2943641, rs6123837, rs662799 and rs1042713; or alternatively, detecting the genotype of rs1801133, rs1052700 and rs2605100, and the detection of the genotype of one, two, three, four, five, six or seven of the SNPs rs1800544, rs3813929, rs10182181, rs2943641, rs6123837, rs662799 and rs1042713; or alternatively, detecting the genotype of rs1801133, rs1052700, rs2605100 and rs3123554, and the detection of the genotype of one, two, three, four, five, six or seven of the SNPs rs1800544, rs3813929, rs10182181, rs2943641, rs6123837, rs662799 and rs1042713; or alternatively, detecting the genotype of rs1801133, rs1052700, rs2605100, rs3123554 and rs10767664, and the detection of the genotype of one, two, three, four, five, six or seven of the SNPs rs1800544, rs3813929, rs10182181, rs2943641, rs6123837, rs662799 and rs1042713; alternatively, detecting the genotype of rs1801133, rs1052700, rs2605100, rs3123554, rs10767664 and rs659366, and the detection of the genotype of one, two, three, four, five, six or seven of the SNPs rs1800544, rs3813929, rs10182181, rs2943641, rs6123837, rs662799 and rs1042713; or alternatively, detecting the genotype of one, two, three, four, five or six of the SNPs rs1801133, rs1052700, rs2605100, rs3123554, rs10767664 and rs659366, and the detection of the genotype of rs1800544; or alternatively, comprises detecting the genotype of one, two, three, four, five or six of the SNPs rs1801133, rs1052700, rs2605100, rs3123554, rs10767664 and rs659366, and the detection of the genotype of rs1800544 and rs3813929; or alternatively, comprises detecting the genotype of one, two, three, four, five or six of the SNPs rs1801133, rs1052700, rs2605100, rs3123554, rs10767664 and rs659366, and the detection of the genotype of rs1800544, rs3813929 and rs10182181; or alternatively, comprises detecting the genotype of one, two, three, four, five or six of the SNPs rs1801133, rs1052700, rs2605100, rs3123554, rs10767664 and rs659366, and the detection of the genotype of rs1800544, rs3813929, rs10182181 and rs2943641; or alternatively, comprises detecting the genotype of one, two, three, four, five or six of the SNPs rs1801133, rs1052700, rs2605100, rs3123554, rs10767664 and rs659366, and the detection of the genotype of rs1800544, rs3813929, rs10182181, rs2943641 and rs6123837; or alternatively, comprises detecting the genotype of one, two, three, four, five or six of the SNPs rs1801133, rs1052700, rs2605100, rs3123554, rs10767664 and rs659366, and the detection of the genotype of rs1800544, rs3813929, rs10182181, rs2943641, rs6123837 and rs662799; or alternatively, comprises detecting the genotype of one, two, three, four, five or six of the SNPs rs1801133, rs1052700, rs2605100, rs3123554, rs10767664 and rs659366, and the detection of the genotype of rs1800544, rs3813929, rs10182181, rs2943641, rs6123837, rs662799 and rs1042713.

In an embodiment of the second aspect of the invention, the method comprises detecting the genotype of the SNPs of the following list: rs1801133, rs1052700, rs2605100, rs3123554, rs10767664 and rs659366; and the detection of the SNPs of the following list: rs1800544, rs3813929, rs10182181, rs2943641, rs6123837, rs662799 and rs1042713.

In an embodiment of the second aspect of the invention the method comprises detecting one or more of the genotypes selected from the following list: AA of rs1801133, TT of rs1052700, GG of rs2605100, AA or AG of rs3123554, TA of rs10767664, CC of rs659366 and combinations thereof; and/or detecting one or more of the genotypes selected from the following list: GG or GC of rs1800544, CC or TT of rs3813929, AG or GG of rs10182181, TC or CC of rs2943641, GA of rs6123837, GG or AA of rs662799, GA of rs1042713 and combinations thereof.

In an embodiment of the second aspect of the invention the method comprises detecting the genotypes selected from the following list: AA of rs1801133, TT of rs1052700, GG of rs2605100, AA or AG of rs3123554, TA of rs10767664 and CC of rs659366; and detecting the genotypes selected from the following list: GG or GC of rs1800544, CC or TT of rs3813929, AG or GG of rs10182181, TC or CC of rs2943641, GA of rs6123837, GG or AA of rs662799 and GA of rs1042713.

In an embodiment of the second aspect of the invention the method comprises detecting the genotypes selected from the following list: GG or GA of rs1801133, AA or AT of rs1052700, AA or AG of rs2605100, GG of rs3123554, TT or AA of rs10767664, CT or TT of rs659366 and combinations thereof; and detecting the genotypes selected from the following: CC of rs1800544, CT of rs3813929, AA of rs10182181, TT of rs2943641, GG or AA of rs6123837, GA of rs662799, GG or AA of rs1042713 and combinations thereof.

In an embodiment of the second aspect of the invention the method comprises detecting the genotypes selected from the following list: GG or GA of rs1801133, AA or AT of rs1052700, AA or AG of rs2605100, GG of rs3123554, TT or AA of rs10767664, and CT or TT of rs659366; and detecting the genotypes selected from the following list: CC of rs1800544, CT of rs3813929, AA of rs10182181, TT of rs2943641, GG or AA of rs6123837, GA of rs662799, and GG or AA of rs1042713.

In an embodiment of the second aspect of the invention, wherein when one or more of the following genotypes are present it is indicative of decision or recommendation of the MHP diet: AA of rs1801133, TT of rs1052700, GG of rs2605100, AA or AG of rs3123554, TA of rs10767664, CC of rs659366 and combinations thereof; whereas, when one or more of the following genotypes are present, it is indicative of decision or recommendation of the LF diet: GG or GC of rs1800544, CC or TT of rs3813929, AG or GG of rs10182181, TC or CC of rs2943641, GA of rs6123837, GG or AA of rs662799, GA of rs1042713 and combinations thereof.

In an embodiment of the second aspect of the invention, wherein when one, two, three, four, five or six of the following genotypes are present it is indicative of decision or recommendation of the MHP diet: AA of rs1801133, TT of rs1052700, GG of rs2605100, AA or AG of rs3123554, TA of rs10767664, CC of rs659366 and combinations thereof; whereas, when one, two, three, four, five, six or seven of the following genotypes are present, it is indicative of decision or recommendation of the LF diet: GG or GC of rs1800544, CC or TT of rs3813929, AG or GG of rs10182181, TC or CC of rs2943641, GA of rs6123837, GG or AA of rs662799, GA of rs1042713 and combinations thereof.

In another embodiment of the second aspect of the invention, when the genotype AA of rs1801133 is detected; or alternatively, when AA of rs1801133 and TT of rs1052700 are detected; or alternatively, when AA of rs1801133, TT of rs1052700 and GG of rs2605100 are detected; or alternatively, when AA of rs1801133, TT of rs1052700, GG of rs2605100 and AA or AG of rs3123554 are detected; or alternatively, when AA of rs1801133, TT of rs1052700, GG of rs2605100, AA or AG of rs3123554 and TA of rs10767664 are detected; or AA of rs1801133, TT of rs1052700, GG of rs2605100, AA or AG of rs3123554, TA of rs10767664 and CC of rs659366 are detected; it is indicative of decision or recommendation of the MHP diet; whereas when the genotype GG or GC of rs1800544 is detected; or alternatively, when GG or GC of rs1800544 and CC or TT of rs3813929 are detected; or alternatively, when GG or GC of rs1800544, CC or TT of rs3813929 and AG or GG of rs10182181 are detected; or alternatively, when GG or GC of rs1800544, CC or TT of rs3813929, AG or GG of rs10182181 and TC or CC of rs2943641 are detected; or alternatively, when GG or GC of rs1800544, CC or TT of rs3813929, AG or GG of rs10182181, TC or CC of rs2943641 and GA of rs6123837 are detected; or alternatively, when GG or GC of rs1800544, CC or TT of rs3813929, AG or GG of rs10182181, TC or CC of rs2943641, GA of rs6123837 and GG or AA of rs662799; or alternatively, when GG or GC of rs1800544, CC or TT of rs3813929, AG or GG of rs10182181, TC or CC of rs2943641, GA of rs6123837, GG or AA of rs662799 and GA of rs1042713 are detected; it is indicative of decision or recommendation of the LF diet.

In an embodiment of the second aspect of the invention, when the following genotypes are present it is indicative of decision or recommendation of the MHP diet: AA of rs1801133, TT of rs1052700, GG of rs2605100, AA or AG of rs3123554, TA of rs10767664 and CC of rs659366; whereas, when the following genotypes are present, it is indicative of decision or recommendation of the LF diet: GG or GC of rs1800544, CC or TT of rs3813929, AG or GG of rs10182181, TC or CC of rs2943641, GA of rs6123837, GG or AA of rs662799 and GA of rs1042713.

In another embodiment of the second aspect of the invention, when one or more (1, 2, 3, 4, 5 or 6) of the genotypes selected from the following list is detected, it is indicative of no decision or no recommendation of MHP diet: GG or GA of rs1801133, AA or AT of rs1052700, AA or AG of rs2605100, GG of rs3123554, TT or AA of rs10767664, CT or TT of rs659366 and combinations thereof. Whereas when one or more (2, 3, 4, 5, 6 or 7) of the genotypes selected from the following list is detected, it is indicative of no decision or no recommendation of the LF diet: CC of rs1800544, CT of rs3813929, AA of rs10182181, TT of rs2943641, GG or AA of rs6123837, GA of rs662799, GG or AA of rs1042713 and combinations thereof.

In another embodiment of the second aspect of the invention, when the genotype GG or GA of rs1801133 is detected; or alternatively, when GG or GA of rs1801133 and AA or AT of rs1052700 are detected; or alternatively, when GG or GA of rs1801133, AA or AT of rs1052700 and AA or AG of rs2605100 are detected; or alternatively, when GG or GA of rs1801133, AA or AT of rs1052700, AA or AG of rs2605100 and GG of rs3123554 are detected; or alternatively, when GG or GA of rs1801133, TT of rs1052700, AA or AG of rs2605100, GG of rs3123554 and TT or AA of rs10767664 are detected; or alternatively, when GG or GA of rs1801133, AA or AT of rs1052700, AA or AG of rs2605100, GG of rs3123554, TT or AA of rs10767664 and CT or TT of rs659366 are detected; it is indicative of no decision or no recommendation of MHP diet. Whereas, when CC of rs1800544 is detected; or alternatively, when CC of rs1800544 and CT of rs3813929 are detected; or alternatively, when CC of rs1800544, CT of rs3813929 and AA of rs10182181 are detected; or alternatively, when CC of rs1800544, CT of rs3813929, AA of rs10182181 and TT of rs2943641 are detected; or alternatively, when CC of rs1800544, CT of rs3813929, AA of rs10182181, TT of rs2943641 and GG or AA of rs6123837 are detected; or alternatively, when CC of rs1800544, CT of rs3813929, AA of rs10182181, TT of rs2943641, GG or AA of rs6123837 and GA of rs662799 are detected; or alternatively, when CC of rs1800544, CT of rs3813929, AA of rs10182181, TT of rs2943641, GG or AA of rs6123837, GA of rs662799, GG or AA of rs1042713 are detected, it is indicative of no decision or no recommendation of LF diet.

In an embodiment of the first and the second aspects of the invention, optionally in combination with any embodiments above or below, the *in vitro* methods further comprise determining a genetic risk score of percentage of BMI loss.

"Genetic risk score (GRS)" or "genetic score" is to be understood as a risk score in which genetic parameters (the genetic variants of the present invention) are taken in consideration when evaluating a particular level of risk. In the present invention, a particular genotype of an SNP is given a value of "0" when it is a non-risk genotype, that is, the subject with said genotype following a specific diet losses % of BMI; whereas, a particular genotype of an SNP is given a value of "1" when it is a risk genotype, that is, a genotype associated with lower % of BMI loss (the subject with said genotype following the specific diet losses less % of BMI). The GRS can be unweighted (uGRS) or weighted (wGRS). The uGRS is calculated by adding the number of each genotypes. The wGRS is defined as weighted sums of risk alleles of the SNPS. wGRS is calculated by multipliying the number obtained of risk genotypes for their corresponding effect sizes as described in Hung CF et al (Hung et al. 2015) and then adding the products.

In an embodiment of the first and second aspects of the invention, the *in vitro* method further comprises considering one or more of the clinical parameters and/or features of the subject selected form age, baseline energy intake, gender, weight, BMI, total body fat, visceral fat, waist circumference, blood pressure, systolic blood pressure, diastolic blood reassure, triglyceride level, hypercholesterolemia, total cholesterol levels high-density lipoprotein cholesterol level, low-density lipoprotein cholesterol triglycerides level, triglyceride-glucose index, energy dietary intake per day, physical activity, diabetes status, hypertension and combinations thereof.

In an embodiment of the first and second aspects of the invention, the method considers age for predicting the % of BMI loss of the MHP diet, or for deciding or recommending the MHP diet; and considers baseline energy intake for predicting the % of BMI loss of the LF diet, or for the deciding or recommending the LF diet.

In an embodiment of the first and the second aspects of the invention, optionally in combination with any embodiments above or below, the *in vitro* methods further comprise and obtaining a predictive value.

The "predictive value" as used herein, refers to a factor that is derived from the genetic risk score and the clinical parameters and/or features. The predictive factor can be calculated by any multiple linear regression models known by the skilled person. The predictive value can be calculated by using the least-angle regression (LARS) (Efron B, Hastie T, Johnstone I, Tibshirani R. Least angle regression. Ann Statist. 2004;32(2):407-499.), best subset regression procedure (BSRP) (Lindsey C, Sheather S. Variable selection in linear regression. Stata Journal. 2010;10(4): 650-669.) or bootstrapping stepwise method (BSM) (Austin PC, Tu JV. Bootstrap Methods for Developing Predictive Models. Am Stat. 2004; 58(2):131-137.).

In a particular embodiment of the second aspect of the invention, the "predictive value" as used herein, refers to a value that is derived from the genetic risk score and the age in the case of the MHP diet, or alternatively is derived from the genetic risk score and the baseline energy intake for the LF diet. In another embodiment the predictive value derived from the genetic risk score and the age in the case of the MHP diet, and derived from the genetic risk score and the baseline energy intake for the LF, is calculated by using the least-angle regression (LARS), best subset regression procedure (BSRP) or bootstrapping stepwise method (BSM) methods.

In an embodiment of the first and second aspects of the invention, the method further comprises comparing statistically the predictive value of % of BMI loss obtained for the MHP diet as defined in the invention and for the LF diet as defined in the present invention.

In a more particular embodiment or the second aspect of the invention, if after the statistical comparison the subject is considered at risk of non-reduction of % of BMI using diet MHP as defined in the invention, then the LF diet as defined in the invention is decided or recommended; whereas if the subject is considered at risk of non-reduction of % of BMI using diet LF as defined in the invention, then the MHP diet as defined in the invention is decided or recommended; whereas when the predictive value is similar to both diets (MHP and LF diets), then any of them can be decided or recommended; and whereas if the subject is considered at risk of non-reduction of % of BMI using diet LF and MHP diet as defined in the invention, then none of them are recommended or decided.

In an embodiment of the first and the second aspects of the invention, the diet is an energy-restricted diet of at least 1200 kcal/day.

In an embodiment of the first and the second aspects of the invention, the patient does not follow an increase in the level of physical activity.

In an embodiment of the first and the second aspects of the invention, the diet is an energy-restricted diet of at least 1200 kcal/day and wherein the patient does not follow an increase in the level of physical activity.

In an embodiment of the first and the second aspects of the invention, the patient has a BMI ≥ 25 kg/m² and <30 kg/m² or, alternatively, wherein the patient has a BMI ≥ 30 kg/m² and <40 kg/m², or, alternatively, wherein the patient has a BMI ≥ 40 kg/m².

The third aspect of the invention refers to the use of means for detecting the genotypes as defined in the first or the second aspect of the invention in a biological sample from a subject for the *in vitro* prediction of the reduction of the % BMI in response to f a diet of the first aspect of the invention or the *in vitro* method for decision or recommendation of a patient to follow an energy-restricted diet as defined of the second aspect of the invention.

In an embodiment of the third aspect of the invention, the means comprise for example, primers, probes buffers, salts and enzymes useful in the detection of the genotypes of the present invention.

In another embodiment of the third aspect of the invention, the means form part of a kit.

In an embodiment of the third aspect of the invention, the subject has a BMI of at least 25 kg/m².

The fourth aspect of the invention refers to the use of at least one of the following SNPs for the *in vitro* prediction of the reduction of the % of BMI in response to a diet as defined in the first aspect of the invention or the *in vitro* method for decision or recommendation of a patient to follow an energy-restricted diet as defined in the second aspect of the invention, which is selected from the following list: rs1801133, rs1052700, rs2605100, rs3123554, rs10767664, rs659366, rs1800544, rs3813929, rs10182181, rs2943641, rs6123837, rs662799, rs1042713 and combinations thereof.

In an embodiment of the fourth aspect of the invention, the use is of one or more (one, two, three, four, five or six) SNPs selected from the following list: rs1801133, rs1052700, rs2605100, rs3123554, rs10767664 and rs659366; and one or more (one, two, three, four, five, six or seven) SNPs selected from the following list: rs1800544, rs3813929, rs10182181, rs2943641, rs6123837, rs662799, rs1042713 and combinations thereof.

In an embodiment of the fourth aspect of the invention, the use is of rs1801133, in combination with one, two, three, four, five, six or seven of the SNPs rs1800544, rs3813929, rs10182181, rs2943641, rs6123837, rs662799 and rs1042713; or alternatively, the use is of rs1801133 and rs1052700, in combination with one, two, three, four, five, six or seven of the SNPs rs1800544, rs3813929, rs10182181, rs2943641, rs6123837, rs662799 and rs1042713; or alternatively, the use is of rs1801133, rs1052700 and rs2605100, in combination with one, two, three, four, five, six or seven of the SNPs rs1800544, rs3813929, rs10182181, rs2943641, rs6123837, rs662799 and rs1042713; or alternatively, the use is of rs1801133, rs1052700, rs2605100 and rs3123554, in combination with one, two, three, four, five, six or seven of the SNPs rs1800544, rs3813929, rs10182181, rs2943641, rs6123837, rs662799 and rs1042713; or alternatively, the use is of rs1801133, rs1052700, rs2605100, rs3123554 and rs10767664, in combination with one, two, three, four, five, six or seven of the SNPs rs1800544, rs3813929, rs10182181, rs2943641, rs6123837, rs662799 and rs1042713; or alternatively, the use is of rs1801133, rs1052700, rs2605100, rs3123554, rs10767664 and rs659366, in combination with one, two, three, four, five, six or seven of the SNPs rs1800544, rs3813929, rs10182181, rs2943641, rs6123837, rs662799 and rs1042713; or alternatively, the use is of one, two, three, four, five or six of the SNPs rs1801133, rs1052700, rs2605100, rs3123554, rs10767664 and rs659366, in combination with rs1800544; or alternatively, the use is of one, two, three, four, five or six of the SNPs rs1801133, rs1052700, rs2605100, rs3123554, rs10767664 and rs659366, in combination with rs1800544 and rs3813929; or alternatively, the use is of one, two, three, four, five or six of the SNPs rs1801133, rs1052700, rs2605100, rs3123554, rs10767664 and rs659366, in combination with rs1800544, rs3813929 and rs10182181; or alternatively, the use is of one, two, three, four, five or six of the SNPs rs1801133, rs1052700, rs2605100, rs3123554, rs10767664 and rs659366, in combination with rs1800544, rs3813929, rs10182181 and rs2943641; or alternatively, the use is of one, two, three, four, five or six of the SNPs rs1801133, rs1052700, rs2605100, rs3123554, rs10767664 and rs659366, in combination with rs1800544, rs3813929, rs10182181, rs2943641 and rs6123837; or alternatively, the use is of one, two, three, four, five or six of the SNPs rs1801133, rs1052700, rs2605100, rs3123554, rs10767664 and rs659366 in combination with rs1800544, rs3813929, rs10182181, rs2943641, rs6123837 and rs662799; or alternatively, the use is of one, two, three, four, five or six of the SNPs rs1801133, rs1052700, rs2605100, rs3123554, rs10767664 and rs659366 in combination with rs1800544, rs3813929, rs10182181, rs2943641, rs6123837, rs662799 and s1042713.

In an embodiment of the fourth aspect of the invention, the use is of the SNPs of the following list: rs1801133, rs1052700, rs2605100, rs3123554, rs10767664 and rs659366; in combination with the SNPs of the following list: rs1800544, rs3813929, rs10182181, rs2943641, rs6123837, rs662799 and rs1042713.

The fifth aspect of the invention refers to the use of at least one of the following genotypes for the *in vitro* prediction of the reduction of the % of BMI in response to a diet as defined in the first aspect of the invention or the *in vitro* method for decision or recommendation of a patient to follow an energy-restricted diet as defined in the second aspect of the invention, which is selected from the following list: AA of rs1801133, TT of rs1052700, GG of rs2605100, AA or AG of rs3123554, TA of rs10767664, CC of rs659366, GG or GC of rs1800544, CC or TT of rs3813929, AG or GG of rs10182181, TC or CC of rs2943641, GA of rs6123837, GG or AA of rs662799, GA of rs1042713 and combinations thereof.

An embodiment of the fifth aspect of the invention refers to the use of at least one of the following genotypes for the *in vitro* prediction of the reduction of the % of BMI in response to a diet as defined in the first aspect of the invention or the *in vitro* method for decision or recommendation of a patient to follow an energy-restricted diet as defined in the second aspect of the invention, which is selected from the following list: AA of rs1801133, TT of rs1052700, GG of rs2605100, AA or AG of rs3123554, TA of rs10767664, CC of rs659366 and combinations thereof; and GG or GC of rs1800544, CC or TT of rs3813929, AG or GG of rs10182181, TC or CC of rs2943641, GA of rs6123837, GG or AA of rs662799, GA of rs1042713 and combinations thereof.

Another embodiment of the fifth aspect of the invention refers to the use of the genotype AA of rs1801133; or alternatively, AA of rs1801133 and TT of rs1052700; or alternatively, AA of rs1801133, TT of rs1052700 and GG of rs2605100; or alternatively, AA of rs1801133, TT of rs1052700, GG of rs2605100 and AA or AG of rs3123554; or alternatively, AA of rs1801133, TT of rs1052700, GG of rs2605100, AA or AG of rs3123554 and TA of rs10767664; or alternatively, AA of rs1801133, TT of rs1052700, GG of rs2605100, AA or AG of rs3123554, TA of rs10767664, CC of rs659366; or alternatively, the genotype GG or GC of rs1800544; or alternatively, GG or GC of rs1800544 and CC or TT of rs3813929; or alternatively, GG or GC of rs1800544, CC or TT of rs3813929 and AG or GG of rs10182181; or alternatively, GG or GC of rs1800544, CC or TT of rs3813929, AG or GG of rs10182181 and TC or CC of rs2943641; or alternatively, GG or GC of rs1800544, CC or TT of rs3813929, AG or GG of rs10182181, TC or CC of rs2943641 and GA of rs6123837; or alternatively, GG or GC of rs1800544, CC or TT of rs3813929, AG or GG of rs10182181, TC or CC of rs2943641, GA of rs6123837 and GG or AA of rs662799; or alternatively, GG or GC of rs1800544, CC or TT of rs3813929, AG or GG of rs10182181, TC or CC of rs2943641, GA of rs6123837, GG or AA of rs662799 and GA of rs1042713.

The fifth aspect of the invention can also be read as the use of at least one of the following genotypes for the *in vitro* determination of the prediction of the reduction of the percentage of BMI in response to a diet as defined in the first aspect of the invention or the *in vitro* method for decision or recommendation of a patient to follow an energy-restricted diet as defined in the second aspect of the invention, which is selected from the following list: GG or GA of rs1801133, AA or AT of rs1052700, AA or AG of rs2605100, GG of rs3123554, TT or AA of rs10767664, CT or TT of rs659366, CC of rs1800544, CT of rs3813929, AA of rs10182181, TT of rs2943641, GG or AA of rs6123837, GA of rs662799, GG or AA of rs1042713 and combinations thereof.

The fifth aspect of the invention can also be read as the use of at least one of the following genotypes for the *in vitro* prediction of the reduction of the percentage of BMI in response to a diet as defined in the first aspect of the invention or the *in vitro* method for decision or recommendation of a patient to follow an energy-restricted diet as defined in the second aspect of the invention, which is selected from the following list: GG or GA of rs1801133, AA or AT of rs1052700, AA or AG of rs2605100, GG of rs3123554, TT or AA of rs10767664, CT or TT of rs659366, and combinations thereof; and CC of rs1800544, CT of rs3813929, AA of rs10182181, TT of rs2943641, GG or AA of rs6123837, GA of rs662799, GG or AA of rs1042713 and combinations thereof.

In an embodiment of the fifth aspect of the invention, the use is of the genotype GG or GA of rs1801133; or alternatively, or the use of GG or GA of rs1801133 and AA or AT of rs1052700; or alternatively, the use of GG or GA of rs1801133, AA or AT of rs1052700 and AA or AG of rs2605100; or alternatively, the use of GG or GA of rs1801133, AA or AT of rs1052700, AA or AG of rs2605100 and GG of rs3123554; or alternatively, the use of GG or GA of rs1801133, TT of rs1052700, AA or AG of rs2605100, GG of rs3123554 and TT or AA of rs10767664; or alternatively, the use of GG or GA of rs1801133, AA or AT of rs1052700, AA or AG of rs2605100, GG of rs3123554, TT or AA of rs10767664 and CT or TT of rs659366; or alternatively, the use of CC of rs1800544; or alternatively, the use of CC of rs1800544 and CT of rs3813929; or alternatively, the use of CC of rs1800544, CT of rs3813929 and AA of rs10182181; or alternatively, the use of CC of rs1800544, CT of rs3813929, AA of rs10182181 and TT of rs2943641; or alternatively, the use of CC of rs1800544, CT of rs3813929, AA of rs10182181, TT of rs2943641 and GG or AA of rs6123837; or alternatively, the use of CC of rs1800544, CT of rs3813929, AA of rs10182181, TT of rs2943641, GG or AA of rs6123837 and GA of rs662799; or alternatively, the use of CC of rs1800544, CT of rs3813929, AA of rs10182181, TT of rs2943641, GG or AA of rs6123837, GA of rs662799 and GG or AA of rs1042713.

The sixth aspect of the invention refers to a method of treatment of a subject with a BMI of at least 25 kg/m² with an energy-restricted diet selected from a diet wherein the total energy (E) intake from lipids is 20-25% (an LF diet); or, alternatively, a diet wherein the E from proteins is 25-35% (MHP diet), comprising detecting in an isolated sample of the subject the genotype of one or more single nucleotide polymorphisms (SNPs) selected from the following list: rs1801133 (MTHFR), rs1052700 (PLIN1), rs2605100 (LYPLAL1), rs3123554 (CNR2), rs10767664 (BDNF), rs659366 (UCP2) and combinations thereof; and the detection of the genotype of one or more SNPs selected from the following list: rs1800544 (ADRA2A), rs3813929 (HTR2C), rs10182181 (ADCY3), rs2943641 (IRS1), rs6123837 (GNAS), rs662799 (APOA5), rs1042713 (ADRB2) and combinations thereof; wherein the subject is considered at risk of non-% of BMI loss using LF diet, then is treated using MHP diet, whereas, when the subject is considered at risk of non-% of BMI loss using MHP diet, then is treated using LF diet.

In an embodiment of the sixth aspect of the invention, the LF diet is a LF diet wherein the total E intake from carbohydrates is 55-65%, E from proteins is 15-20% and E from lipids is 20-25%; or/and the MHP diet is a MHP diet wherein the E from carbohydrates is 40-50%, E from proteins is 25-35% and E from lipids is 28-35%.

In an embodiment of the sixth aspect of the invention, the LF diet is a LF diet wherein the total E intake from carbohydrates is 60%, E from proteins is 18% and E from lipids is 22%; or/and the MHP diet is a MHP diet wherein the E from carbohydrates is 40%, E from proteins is 30% and E from lipids is 30%.

In the present invention all embodiments of the first and second aspects of the invention are also embodiments of the sixth aspect of the invention.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

### Example 1: Prediction of percentage of BMI loss in MHP and LF diets

### Materials and methods:

### Study participants:

This clinical trial (No. Identifier: NCT02737267) enrolled 353 Spanish adults of self-reported Caucasian ancestry, who presented overweight (BMI 25-29.9 kg/m²) or obesity (BMI 30-40 kg/m²). Unrelated volunteers were recruited at the Center for Nutrition Research of the University of Navarra in the city of Pamplona, Navarra, Spain. Major exclusion criteria included a clinical history of cardiovascular disease, type 1 diabetes, or type 2 diabetes treated with insulin; pregnant or lactating women; individuals reporting weight change (> 3 kg) within the 3 months before the study; and unstable medication for weight control, or hyperlipidemia and hypertension treatments. Subjects with relevant alcohol consumption (> 40 g of ethanol/d in men and > 20 g of ethanol/d in women) were also excluded from the trial. All research procedures were implemented in agreement with the ethical principles of the 2013 Helsinki Declaration (WMA, 2013). Each participant voluntarily provided a written informed consent before inclusion in the study. The study protocol was specifically approved by the Research Ethics Committee of the University of Navarra (Ref. 132/2015).

### Anthropometric and blood pressure measurements:

Anthropometric measurements including body weight, height, and waist circumference (WC) were collected by trained nutritionists following conventional validated methods (Lopez-Legarrea P, 2013; de la Iglesia R, 2014). BMI was calculated as the ratio between kilograms by squared meters (kg/m2). Total body fat (TFAT, %) and visceral fat (VFAT, g) contents were determined by dual-energy x-ray absorptiometry as described by the supplier (Lunar Prodigy, software version 6.0, Madison, WI, USA). Systolic and diastolic blood pressures were quantified with an automated sphygmomanometer according to the standardized criteria of the World Health Organization (WHO) and the International Society of Hypertension (Whitworth JA, 2004).

### Biochemical tests:

Fasting blood samples were drawn by venipuncture, and serum was processed in an automatic analyzer (Pentra C200, HORIBA Medical) using appropriate kits provided by the company. Blood tests included glucose, total cholesterol (TC), high-density lipoprotein cholesterol (HDL-c), and triglycerides (TG). Low-density lipoprotein cholesterol (LDL-c) was calculated applying the Friedewald equation: LDL-c = TC - HDL-c - triglycerides/5 (Tremblay AJ, 2004). In addition, the triglyceride-glucose (TyG) index was estimated as described elsewhere (Navarro-González D, 2016).

### Habitual dietary intake and physical activity assessments:

Habitual dietary intake at baseline was evaluated with a validated semi-quantitative food frequency questionnaire of 137 food items (Martin-Moreno JM, 1993; de la Fuente-Arrillaga C, 2010). Enrolled participants were asked to report how frequent (daily, weekly, monthly or never) they had consumed each food item during the previous year. An *ad hoc* computer program was used to translate food consumption into energy and nutrient intake based on the information available from standard Spanish food composition tables (Moreiras O, 2013).

The level of physical activity of each participant at baseline was estimated using a validated questionnaire collecting information about 17 specific activities (Martínez-González MA, 2005). The volume of activity was expressed as metabolic equivalents (METS), which were computed by assigning a multiple of resting metabolic rate to each activity that were further multiplied by the time spent in each of the registered activities, as detailed elsewhere (Basterra-Gortari FJ, 2009).

### Nutritional intervention:

A four-month nutritional intervention was conducted involving two energy-restricted diets (30% restriction of the total energy expenditure) with different macronutrient distribution. A low fat (LF) diet was designed to provide 60% of total energy (E) intake from carbohydrates, 18% E from proteins, and 22% E from lipids, as designed in the Nutrient-Gene Interactions in Human Obesity trial (Handjieva-Darlenska T, 2012). On the other hand, a moderately high-protein (MHP) diet supplied 40% E from carbohydrates, 30% E from proteins, and 30% E from lipids based on the Metabolic Syndrome Reduction in Navarra, Spain study criteria (de la Iglesia R, 2014). Individual energy requirements of each participant at baseline were estimated by calculating the energy expenditure at rest and during physical activity, as previously reported (Ramos-Lopez O, 2018a). The energy content of LF and MHP diets was no less than 1200 kcal/d.

Participants were randomly assigned to one of the two dietary interventions with a specific algorithm using the online MATLAB software (http://www.mathworks.com) and stratified by sex, age groups (<45 y and ≥45 y) and BMI (25-29.9 kg/m2 and 30-40 kg/m2). Both LF and MHP diets were designed on the basis of a food exchange system (Goni L, 2016). For this purpose, participants received detailed information from trained dietitians concerning portion sizes, feeding schedules, and food preparation techniques. Adherence to the diet was evaluated based on dietitian's criteria using the following scale: 3 = Very good adherence (continuous follow up); 2 = Good adherence (occasionally exceeded from recommendations); 1 = Regular adherence (follow up during weekdays but no in weekend); and 0 = Poor adherence (failure to follow the diet at any time). This test was applied at two times, in the middle (8th week) and at the end (16th week) of the intervention. In addition, dietitians conducted motivational telephone calls with each participant during the whole period of the nutritional intervention to guarantee adherence to the dietary advice.

### SNPs selection and genotyping:

This method allowed the detection of 97 SNPs near or within 59 genes.

Buccal samples were collected from each participant with a liquid-based kit (ORAcollect-DNA, OCR-100, DNA genotek Inc, Ottawa, Canada). High-quality genomic DNA was isolated using the Maxwell® 16 Buccal Swab LEV DNA Purification Kit in the Maxwell® 16 Instrument (Promega Corp, Madison, WI, USA). A customized panel of primers to amplify the regions containing the selected SNPs was designed using the "on line" application of Thermo Fisher AmpliSeq Designer (ID n°. IAD75470, Thermo Fisher). Overall, the amplicon average size was 185 bp. The amplicon library for massive sequencing was constructed with the custom-designed panel and the Ion AmpliSeq™ Library Kit 2.0 (Thermo Fisher Scientific Inc, Waltham, MA, USA) according to the manufacturer's protocol.

Genotyping was performed by targeted next generation sequencing in the Ion Torrent PGM™ equipment (Thermo Fisher Scientific Inc, Waltham, MA, USA), as described elsewhere (Ramos-Lopez O, 2018b). Raw data were processed in the Ion Torrent Suite™ Server Version 5.0.4 (Thermo Fisher Scientific Inc, Waltham, MA, USA) using the Homo sapiens (HG19) as the reference genome for the alignment. A custom-designed Bed file was used to locate the SNPs of interest. Genetic variants were identified with the Torrent Variant Caller 5.0 (Thermo Fisher Scientific Inc, Waltham, MA, USA) with a minimum coverage value of 25 sequences (Guo F, 2016). The pilot validation tests resulted in the sequencing of 95 SNPs of the 97 designed. Hardy-Weinberg equilibrium (HWE) for all genetic variants was estimated using the Convert program (Version 1.31) and the Arlequin software (Version 3.0).

### Statistical analyses:

In order to detect a minimum difference of weight loss of approximately 2 kg ± 3.5 kg between dietary groups, the required sample size was estimated at 200 individuals (α = 0.05 and statistical power of 80%). However, considering a potential dropout rate of 30%, it was considered necessary to recruit about 260 subjects. The distribution (normality) of studied variables was screened by the Kolmogorov-Smirnov test. All principal variables including adiposity and metabolic markers were normally distributed (P > 0.05). Quantitative variables were expressed as means ± standard deviations, while qualitative variables were presented as numbers and percentages. Average changes from baseline to the end of both nutritional interventions were adjusted by age and sex using ANCOVA. Statistical differences between sex, age, SNPs, and diets were estimated using t-student and χ² tests.

The prediction of the difference in BMI loss (%) by diet was performed using multiple linear regression models. Three statistical approaches were used to select explanatory models: Least-Angle Regression (LARS) (Efron B, 2004), Best Subset Regression Procedure (BSRP) (Lindsey C, 2010), and bootstrapping stepwise method (BSM) (Austin PC, 2004), which were run in the statistical program STATA 12 (StataCorp LLC, College Station, TX, USA).

For the screening of SNPs to introduce into the models, ANOVA tests were performed in a first step to select the SNPs presenting at least a marginal statistical trend (P <0.10) between genotypes concerning BMI loss. Second, post hoc tests (Bonferroni's and Dunnett's T3) were run to define intergroup differences in order to differentially codify each SNP genotype as 0 (non-risk) and 1 (risk). A risk genotype was defined as the one that was associated with lower BMI loss. Genotypes with similar effects (P > 0.05) were clustered in a single category. In a third step, t-student tests were further applied to confirm statistical differences between the categorized genotype groups (risk vs. non-risk). Then, SNPs showing at least a marginal statistical trend (P <0.10) were selected. In order to avoid instability in the regression model, SNPs with a low prevalence (< 10%) in either genotype category (risk and non-risk) were excluded. To evaluate the combined effects of the previously selected SNPs on BMI loss, unweighted and weighted genetic risk scores (GRS) for each diet were computed. Unweighted GRS for MHP (uGRS1) and LF (uGRS2) diets were calculated by adding the number of risk genotypes at each locus. Instead, weighted GRS for MHP (wGRS1) and LF (wGRS2) diets were constructed by multiplying the number of risk genotypes for their corresponding effect sizes, and then summing the products, as described elsewhere (Hung CF, 2015). Analyses were performed using uGRS and wGRS as continuous variables.

Besides genetic variants, other conventional predictors of personalization were evaluated including age, sex, physical activity (METs), and baseline energy intake (kcal). Candidate models were tested to verify residual's homoscedasticity with the Breusch-Pagan/Cook-Weisberg test (Ou Z, 2015). Moreover, multicollinearity of the predictors (Iacobucci D, 2017) was evaluated using the variance inflation factor (VIF). To select the best multiple linear regression model, a correction for optimistic prediction was performed according to Harrell's bootstrap method (Harrell FE Jr, 1996). Thus, the model with the higher adj. R2 value after applying the correction for optimism was chosen. Furthermore, the confidence interval of each prediction by diet was calculated. Statistical differences were analyzed using Z test using the standard errors of each prediction in order to prescribe the most suitable diet to each individual. Statistical significance was based on a P value < 0.05.

### Results:

A total of 232 participants completed the nutritional intervention. However, 31 participants who objectively presented poor dietary adherence (see nutritional intervention section) were excluded. Thus, a total of 201 subjects were analyzed. The baseline characteristics of these participants according to sex and age categories are shown in table 1. Overall, 70% of participants were women. Thirty-five percent of subjects were overweight (n = 70), whereas 65% (n = 131) presented grade I and grade II obesity according to the BMI classification of the WHO. Expected differences in anthropometry, clinical and biochemical variables between sex and age groups were found. Men were significantly heavier, presented greater adiposity, blood pressure, serum glucose, HDL-c, and TG as well as higher caloric consumption when compared to women. Similarly, subjects older than 47 years had higher adiposity, blood pressure, glucose, and lipid profile than younger individuals (table 1).

**Table 1. Baseline characteristics of the study participants according to sex and age categories.**

| Variable | Men | Women | *P* value^{a} | ≤47 years | >47 years | *P* value^{b} |
|---|---|---|---|---|---|---|
| n= | 60 | 141 | - | 102 | 99 | - |

| *Anthrop.and clinical data* | | | | | | |
|---|---|---|---|---|---|---|
| Weight (kg) | 98.6±11.4 | 83.3±10.9 | **<0.001** | 88.8±13.5 | 86.9±12.6 | 0.309 |
| BMI (kg/m²) | 31.9±3.0 | 31.7±3.6 | 0.694 | 31.2±3.2 | 32.2±3.7 | **0.040** |
| TFAT (%) | 35.0±4.3 | 44.9±4.1 | **<0.001** | 41.2±6.3 | 42.6±5.9 | 0.103 |
| VFAT (kg) | 2.2±0.1 | 1.1±0.5 | **<0.001** | 1.2±0.7 | 1.7±0.9 | **<0.001** |
| WC (cm) | 108.5±0.8 | 99.7±9.7 | **<0.001** | 100.2±10.2 | 104.4±10.0 | **0.004** |
| SBP (mmHg) | 138.7±16.9 | 124.5±16.6 | **<0.001** | 123.7±15.9 | 134.1±18.3 | **<0.001** |
| DBP (mmHg) | 83.7±11.5 | 77.5±10.1 | **<0.001** | 76.5±10.5 | 82.4±10.5 | **<0.001** |

| *Biochem. profile* | | | | | | |
|---|---|---|---|---|---|---|
| Glucose (mg/dL) | 99.0±11.0 | 94.1±9.7 | **0.002** | 92.6±7.5 | 98.7±11.9 | **<0.001** |
| TC (mg/dL) | 223.0±40.4 | 213.4±37.7 | 0.110 | 205.2±38.2 | 227.7±35.9 | **<0.001** |
| HDL-c (mg/dL) | 45.9±7.8 | 59.1±12.8 | **<0.001** | 53.2±13.3 | 57.2±12.5 | **0.028** |
| TG (mg/dL) | 130.8±74.2 | 89.6±38.4 | **<0.001** | 92.7±49.7 | 111.4±58.5 | **0.016** |
| TyG index | 4.67±0.26 | 4.48±0.22 | **<0.001** | 4.47±0.23 | 4.60±0.25 | **<0.001** |

| *Dietary intake*/*day* | | | | | | |
|---|---|---|---|---|---|---|
| Energy (Kcal) | 1475.3±271.7 | 1274.0±224.3 | **<0.001** | 1334.4±270.9 | 1332.6±240.8 | 0.960 |
| Carbohydrate (%E) | 47.3±7.6 | 47.5±9.0 | 0.764 | 47.0±8.8 | 48.0±8.4 | 0.368 |
| Protein (%E) | 24.5±5.1 | 24.2±5.0 | 0.880 | 24.8±5.3 | 23.7±4.7 | 0.102 |
| Fat (%E) | 28.2±4.9 | 28.3±6.0 | 0.793 | 28.2±5.8 | 28.3±5.6 | 0.800 |

| *Lifestyle factor* | | | | | | |
|---|---|---|---|---|---|---|
| Physical activity (METs) | 31.9±23.0 | 20.9±16.0 | **<0.001** | 26.3±20.8 | 21.9±16.8 | 0.098 |

| | | | | | | |
|---|---|---|---|---|---|---|
| Variables were expressed as means ± standard deviations. Anthrop.: anthropometric; BMI: body mass index; TFAT: total body fat; VFAT: visceral fat; WC: waist circumference; SBP: systolic blood pressure; DBP: diastolic blood pressure; Biochem.: biochemical; TC: total cholesterol; HDL-c: high-density lipoprotein cholesterol; TG: triglycerides; TyG index: triglyceride-glucose index; METs: metabolic equivalents. ^{a}Comparison of men and women by *t* student test. ^{b}Comparison of age categories by *t* student test. Bold numbers indicate *P* < 0.05. | | | | | | |

The average values of changes in main variables in response to MHP and LF dietary interventions by sex and age categories were reported (table 2). After adjustments by age and sex, both MHP and LF dietary interventions induced important losses concerning body weight (-8.5 kg vs.-9.1 kg), VF (-463.7 g vs. -489.1 g) and SBP (-12.4 mmHg vs. -10.9 mmHg) as well as substantial decreases in the serum levels of TC (-18.2 mg/dL vs. -22.7 mg/dL) and TG (-19.2 mg/dL vs. -14.2 mg/dL), respectively, with no statistically significant differences between diets in all paired comparisons. On the other hand, relevant differences depending on sex and age were found. The men showed greater reductions in adiposity markers, SBP, HDL-c, TG, and TyG index than women. Also, subjects older than 47 years had higher losses of VFAT, SBP, and glucose than younger subjects (Table 2).

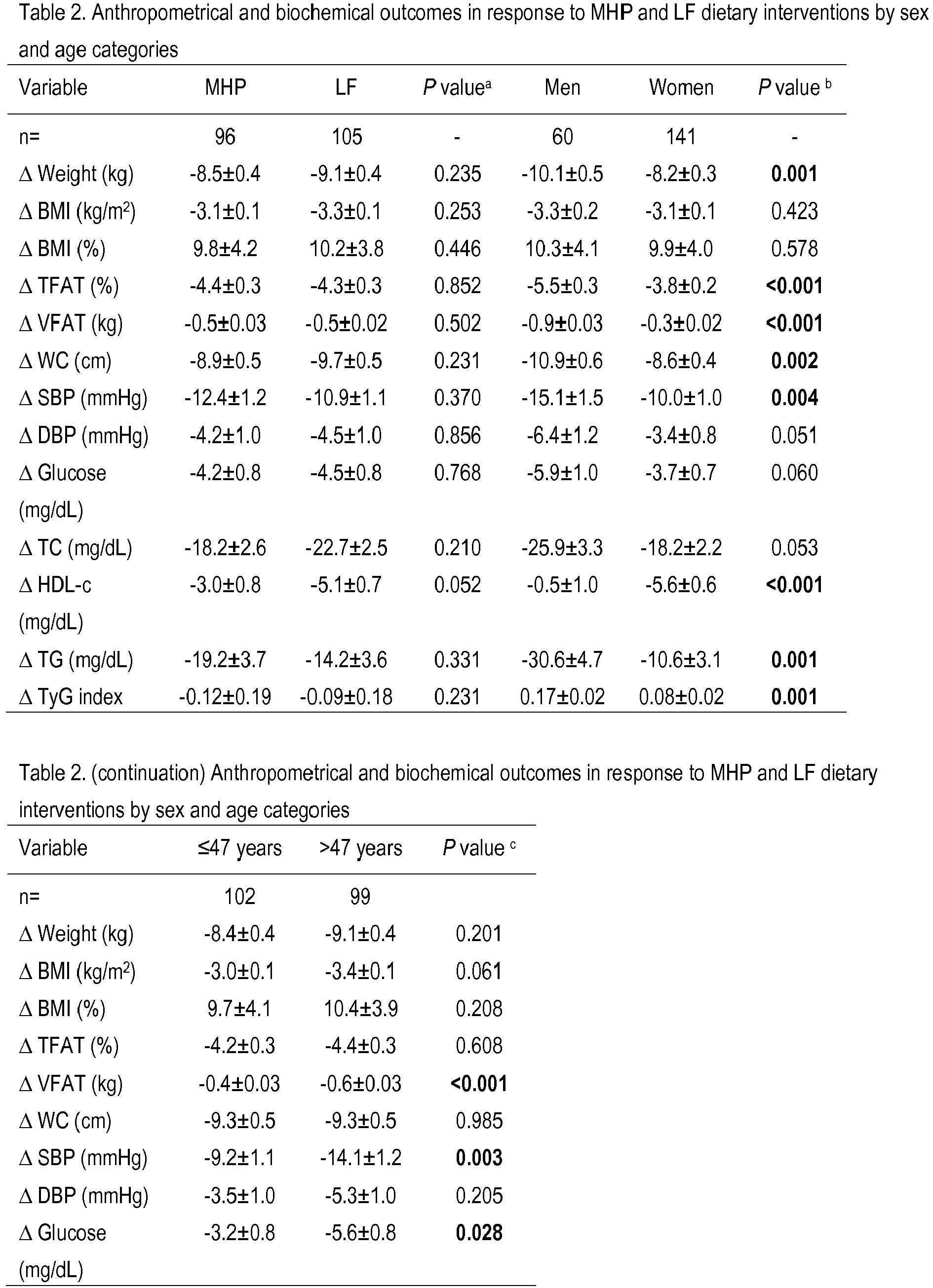

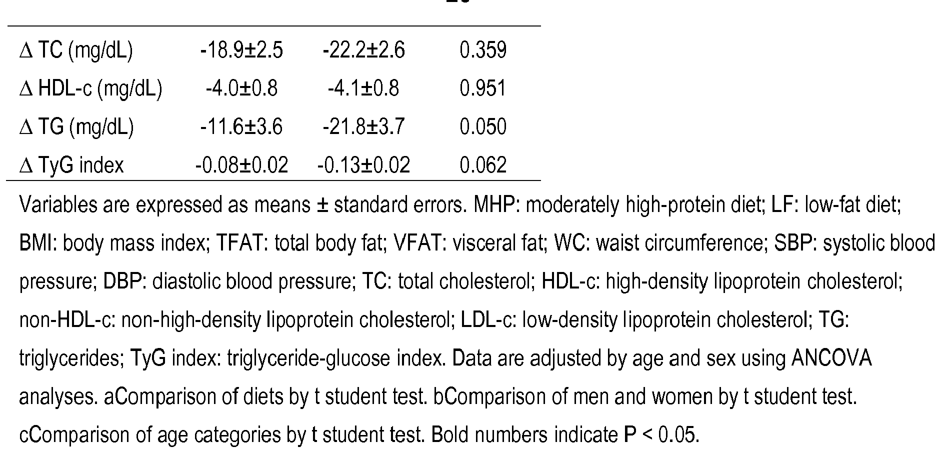

The 95 SNPs were evaluated as indicated in the previous section (genomic information is provided in table 3).

**Table 3. Genomic characteristics of the 95 genotyped SNPs.**

| Gene | SNP | Chromosome position | Alleles | MAF | HWE |
|---|---|---|---|---|---|
| *GNAT2* | rs17024393 | Chr1:110154688 | T/C | 0.019 (C) | 0.087 |
| *MTHFR* | rs1801131 | Chr1:11854476 | T/G | 0.271 (G) | 1.000 |
| *MTHFR* | rs1801133 | Chr1:11856378 | G/A | 0.444 (A) | 0.375 |
| *SEC16B* | rs543874 | Chr1:177889480 | A/G | 0.145 (G) | 1.000 |
| *LYPLAL1* | rs2605100 | Chr1:219644224 | A/G | 0.318 (A) | 0.724 |
| *LYPLAL1* | rs4846567 | Chr1:219750717 | G/T | 0.313 (T) | 0.733 |
| *CNR2* | rs3123554 | Chr1:24196401 | A/G | 0.439 (A) | 1.000 |
| *FAAH* | rs324420 | Chr1:46870761 | C/A | 0.164 (A) | 0.825 |
| *LEPR* | rs8179183/rs1805094 | Chr1:66075952 | G/C | 0.112 (C) | 0.583 |
| *NEGR1* | rs2815752 | Chr1:72812440 | G/A | 0.341 (G) | 0.217 |
| *ABCB11* | rs519887 | Chr2:169780885 | T/C | 0.542 (C) | 0.254 |
| *ABCB11* | rs484066 | Chr2:169782481 | A/T | 0.355 (A) | 0.369 |
| *ABCB11* | rs569805 | Chr2:169782880 | A/T | 0.355 (A) | 0.435 |
| *ABCB11* | rs494874 | Chr2:169789306 | T/C | 0.346 (T) | 0.532 |
| *IRS1* | rs2943641 | Chr2:227093745 | T/C | 0.388 (T) | 1.000 |
| *ADCY3* | rs10182181 | Chr2:25150296 | A/G | 0.491 (A) | 0.160 |
| *ADCY3* | rs713586 | Chr2:25158008 | T/C | 0.481 (T) | 0.206 |
| *TMEM18* | rs2860323 | Chr2:614210 | A/G | 0.140 (A) | 0.813 |
| *TMEM18* | rs2867125 | Chr2:622827 | T/C | 0.145 (T) | 1.000 |
| *TMEM18* | rs13021737 | Chr2:632348 | A/G | 0.150 (A) | 1.000 |
| *PPARG* | rs1801282 | Chr3:12393125 | C/G | 0.117 (G) | 1.000 |
| *PPARG* | rs2959272 | Chr3:12442833 | T/G | 0.523 (T) | 0.482 |
| *PPARG* | rs1386835 | Chr3:12450918 | A/G | NA | <0.001 |
| *PPARG* | rs709158 | Chr3:12463176 | A/G | 0.341 (G) | 0.873 |
| *PPARG* | rs1175540 | Chr3:12465243 | C/A | 0.346 (A) | 1.000 |
| *PPARG* | rs1175544 | Chr3:12467044 | C/T | 0.327 (T) | 0.526 |
| *PPARG* | rs1797912 | Chr3:12470239 | A/C | 0.374 (C) | 0.878 |
| *ETV5* | rs1516725 | Chr3:185824004 | T/C | 0.173 (T) | 0.436 |
| *ETV5* | rs9816226 | Chr3:185834499 | A/T | 0.234 (A) | 0.439 |
| *SLC39A8* | rs13107325 | Chr4:103188709 | C/A/T | 0.084 (T) | 0.606 |
| *FABP2* | rs1799883 | Chr4:120241902 | T/C | 0.271 (T) | 0.385 |
| *UCP1* | rs6536991 | Chr4:141481581 | T/C | 0.322 (C) | 0.099 |
| *UCP1* | rs12502572 | Chr4:141485134 | G/A | 0.621 (G) | 0.492 |
| *UCP1* | rs1800592 | Chr4:141493961 | T/C | 0.257 (C) | 0.338 |
| *PPARGC1A* | rs8192678 | Chr4:23815662 | C/T | 0.388 (T) | 0.199 |
| *GNPDA2* | rs10938397 | Chr4:45182527 | A/G | 0.374 (G) | 0.051 |
| *CLOCK* | rs1801260 | Chr4:56301369 | A/G | 0.294 (G) | 0.369 |
| *PPM1K* | rs1440581 | Chr4:89226422 | T/C | 0.430 (T) | 0.776 |
| *ADRB2* | rs1042713 | Chr5:148206440 | G/A | 0.383 (A) | 0.0678 |
| *ADRB2* | rs1042714 | Chr5:148206473 | G/C | 0.425 (G) | <0.001 |
| *CPEB4* | rs6861681 | Chr5:173362458 | G/A | 0.206 (A) | 0.391 |
| *TNFA* | rs1800629 | Chr6:31543031 | G/A | 0.145 (A) | 1.000 |
| *NUDT3* | rs206936 | Chr6:34302869 | A/G | 0.210 (G) | 0.444 |
| *TFAP2B* | rs987237 | Chr6:50803050 | A/G | 0.145 (G) | 0.609 |
| *TFAP2B* | rs2207139 | Chr6:50845490 | A/G | 0.131 (G) | 0.427 |
| *LEP* | rs7799039 | Chr7:127878783 | G/A | 0.388 (A) | 0.887 |
| *LEP* | rs4731426 | Chr7:127882070 | G/C | 0.561 (C) | 0.543 |
| *LEP* | rs2071045 | Chr7:127892980 | T/C | 0.313 (C) | 0.849 |
| *NFE2L3* | rs1055144 | Chr7:25871109 | C/T | 0.192 (T) | 0.306 |
| *ADRB3* | rs4994 | Chr8:37823798 | A/G | 0.061 (G) | 0.371 |
| *ADRA2A* | rs1800544 | Chr10:112836503 | G/C | 0.729 (C) | 0.611 |
| *ACSL5* | rs2419621 | Chr10:114135013 | C/T | 0.276 (T) | 0.149 |
| *TCF7L2* | rs7903146 | Chr10:114758349 | C/T | 0.397 (T) | 1.000 |
| *TCF7L2* | rs12255372 | Chr10:114808902 | G/T | 0.374 (T) | 0.651 |
| *ANKK1* | rs1800497 | Chr11:113270828 | G/A | 0.145 (A) | 1.000 |
| *APOA5* | rs662799 | Chr11:116663707 | G/A | 0.093 (G) | 1.000 |
| *BDNF* | rs6265 | Chr11:27679916 | C/T | 0.210 (T) | 1.000 |
| *BDNF* | rs11030104 | Chr11:27684517 | A/G | 0.238 (G) | 0.556 |
| *BDNF* | rs10767664 | Chr11:27725986 | T/A | 0.266 (T) | 0.212 |
| *CRY2* | rs11605924 | Chr11:45873091 | A/C | 0.472 (C) | 0.888 |
| *MTCH2* | rs10838738 | Chr11:47663049 | A/G | 0.360 (G) | 0.074 |
| *UCP2* | rs660339 | Chr11:73689104 | G/A | 0.364 (A) | 0.883 |
| *UCP2* | rs659366 | Chr11:73694754 | C/T | 0.332 (T) | 0.756 |
| *UCP3* | rs2075577 | Chr11:73715542 | G/A | 0.579 (G) | 0.887 |
| *UCP3* | rs2734827 | Chr11:73716277 | G/A | 0.308 (A) | 1.000 |
| *UCP3* | rs1685325 | Chr11:73717025 | T/C | 0.598 (T) | 0.889 |
| *UCP3* | rs2075576 | Chr11:73717121 | C/T | 0.164 (T) | 0.649 |
| *UCP3* | rs1800006 | Chr11:73717254 | A/G | 0.173 (G) | 0.368 |
| *UCP3* | rs1800849 | Chr11:73720165 | G/A | 0.164 (A) | 0.642 |
| *STK33* | rs4929949 | Chr11:8604593 | T/C | 0.481 (C) | 0.325 |
| *MTNR1B* | rs10830963 | Chr11:92708710 | C/G | 0.276 (G) | 0.180 |
| *ALOX5AP* | rs4769873 | Chr13:31312689 | C/T | 0.079 (T) | 0.713 |
| *PLIN1* | rs1052700 | Chr15:90208310 | A/T | 0.308 (T) | 0.870 |
| *PLIN1* | rs894160 | Chr15:90211823 | C/T | 0.280 (T) | 1.000 |
| *PLIN1* | rs2289487 | Chr15:90217096 | C/T | 0.654 (T) | 0.749 |
| *SH2B1* | rs7498665 | Chr16:28883241 | A/G | 0.294 (G) | 0.064 |
| *SH2B1* | rs7359397 | Chr16:28885659 | C/T | 0.290 (T) | 0.062 |
| *FTO* | rs1558902 | Chr16:53803574 | T/A | 0.393 (A) | 0.186 |
| *FTO* | rs1121980 | Chr16:53809247 | G/A | 0.411 (A) | 0.375 |
| *FTO* | rs17817449 | Chr16:53813367 | T/G | 0.374 (G) | 0.762 |
| *FTO* | rs8050136 | Chr16:53816275 | C/A | 0.374 (A) | 0.761 |
| *FTO* | rs3751812 | chr16:53818460 | G/T | 0.374 (T) | 0.651 |
| *FTO* | rs9939609 | Chr16:53820527 | T/A | 0.374 (A) | 0.881 |
| *AANAT* | rs12452844 | Chr17:74459243 | G/A | 0.276 (A) | 0.192 |
| *NPC1* | rs1805081 | Chr18:21140432 | T/C | 0.355 (C) | 0.273 |
| *MC4R* | rs6567160 | Chr18:57829135 | T/C | 0.257 (C) | 0.136 |
| *MC4R* | rs571312 | Chr18:57839769 | C/A | 0.257 (A) | 0.127 |
| *MC4R* | rs17782313 | Chr18:57851097 | T/C | 0.257 (C) | <0.001 |
| *MC4R* | rs17066866 | Chr18:58055619 | A/T | 0.009 (T) | 1.000 |
| *TNFRSF11A* | rs17069904 | Chr18:60032949 | G/A | 0.126 (A) | 0.701 |
| *QPCTL* | rs2287019 | Chr19:46202172 | C/T | 0.173 (T) | 0.016 |
| *CTNNBL1* | rs6013029 | Chr20:36399580 | G/T | 0.107 (T) | 0.648 |
| *GNAS* | rs6123837 | Chr20:57465571 | G/A | 0.388 (A) | 0.883 |
| *HTR2C* | rs3813929 | ChrX:113818520 | C/T | 0.156 (T) | <0.001 |
| *AGTR2* | rs11091046 | ChrX:115305126 | A/C | 0.412 (A) | 0.104 |

| | | | | | |
|---|---|---|---|---|---|
| MAF: Minor Allele frequency; HWE: Hardy-Weinberg equilibrium; NA: not available. MAF are reported concerning Iberian (Spanish) population. HWE is expressed as *P* value. | | | | | |

A total of 13 SNPs were statistically or marginally associated with BMI loss (%), all of which were different between MHP and LF diets (see table 4). Six SNPs were statistically or marginally associated with BMI loss in the MHP diet, which were located in genes related to one-carbon metabolism (*MTHFR*), lipid breakdown (*LYPLAL1*), appetite (*CNR2*), neuronal cell differentiation (*BDNF*), thermogenesis (*UCP2*), and lipid storage (*PLIN1*). Whereas, BMI loss in the LF diet was influenced by other different seven SNPs mapped to genes implicated in insulin signalling (*IRS1*), odorant detection (*ADCY3*), cardiovascular function (*ADRB2*), neurotransmitter release (*ADRA2A* and *HTR2C*), lipid transport (*APOA5*), and hormone production (*GNAS*). Although rs3813929 (*HTR2C*) did not show a significant statistical trend when analyzed individually, it substantially contributed to explain BMI loss variance in the corresponding multiple linear regression model.

**Table 4. Genotype codifications of single specific SNPs statistically or marginally associated with BMI loss by diets.**

| | Genotypes | | n | | X±SD | | |
|---|---|---|---|---|---|---|---|
| MHP diet | 0 | 1 | 0 | 1 | 0 | 1 | *P* value^{a} |
| rs1801133 (*MTHFR*) | AA | GG+GA | 16 | 80 | -11.8±4.7 | -9.4±4.1 | 0.041 |
| rs2605100 (*LYPLAL1*) | GG | AA+AG | 47 | 49 | -10.7±3.9 | -8.9±4.4 | 0.033 |
| rs3123554 (*CNR2*) | AA+AG | GG | 71 | 25 | -10.3±4.4 | -8.5±3.7 | 0.068 |
| rs10767664 (*BDNF*) | TA | TT+AA | 29 | 67 | -11.1±4.8 | -9.2±3.9 | 0.056 |
| rs659366 (*UCP2*) | CC | CT+TT | 48 | 48 | -10.7±4.2 | -8.9±4.2 | 0.031 |
| rs1052700 (*PLIN1*) | TT | AA+AT | 12 | 84 | -11.8±4.4 | -9.5±4.2 | 0.079 |

| LF diet | | | | | | | |
|---|---|---|---|---|---|---|---|
| rs2943641 (*IRS1*) | TC+CC | TT | 89 | 16 | -10.6±3.8 | -8.16±3.25 | 0.019 |
| rs10182181 (*ADCY3*) | AG+GG | AA | 80 | 25 | -10.7±3.7 | -8.78±3.88 | 0.031 |
| rs1042713 (*ADRB2*) | GA | GG+AA | 59 | 46 | -10.9±3.7 | -9.38±3.94 | 0.048 |
| rs1800544 (*ADRA2A*) | GG+GC | CC | 51 | 54 | -11.4±3.6 | -9.10±3.71 | 0.002 |
| rs662799 (*APOA5*) | GG+AA | GA | 87 | 18 | -10.6±3.8 | -8.54±3.45 | 0.041 |
| rs6123837 (*GNAS*) | GA | GG+AA | 49 | 56 | -11.0±3.6 | -9.57±3.93 | 0.067 |
| rs3813929 (*HTR2C*) | CC+TT | CT | 87 | 18 | -10.5±4.0 | -8.92±2.83 | 0.117 |

| | | | | | | | |
|---|---|---|---|---|---|---|---|
| ^{a}Comparison of means between risk and non-risk genotypes by t student test. Variables are expressed as number of cases and means ± standard deviations. MHP: moderately high-protein diet; LF: low-fat diet. 0 = non-risk genotypes; 1 = risk genotypes. | | | | | | | |

The SNPs rs1801133 (*MTHFR*), rs2605100 (*LYPLAL1*), rs3123554 (*CNR2*), rs10767664 (*BDNF*), rs659366 (*UCP2*) and rs1052700 (*PLIN1*) accounted by differences in BMI loss in the MHP diet. Meanwhile, BMI loss diverged among genotypes of rs2943641 (*IRS1*), rs10182181 (*ADCY3*), rs1042713 (*ADRB2*), rs1800544 (*ADRA2A*), rs662799 (*APOA5*), rs6123837 (*GNAS*), rs3813929 (*HTR2C*). With these SNPs, two specific wGRS (wGRS1 and wGRS2) were constructed as previously mentioned in the methods section and used for further analyses.

BMI loss (%) prediction by diet was evaluated through multiple linear regression models using genetic (single SNPs and wGRS) and phenotypical variables (age, sex, physical activity level, and baseline energy intake). The statistical performance of all screened models is presented, indicating the best model with the higher adj. R2 value after applying the correction for optimism (tables 4 and 5 below). Higher predictive values of BMI loss variance were found when wGRS for each diet were introduced into the models compared to the effect sizes of each individual SNP. Thus, BMI loss in the MHP was best predicted in about 28% (optimism-corrected adj. R2 = 0.279) by wGRS1 and age according to the following formula: -12.8 + 1.0 x wGRS1 [Σ weighted genotype risks] + -0.11 x age [years]. Instead, wGRS2 and baseline energy intake explained approximately 29% (optimism-corrected adj. R2 = 0.287) of BMI loss variance in the LF diet, resulting in the following formula: -10.9 + 1.0 x wGRS2 [Σ weighted genotype risks] + -0.86 x baseline energy intake [1000 kcal]. In addition, correlations of statistically significant predictors of BMI loss by diet were plotted (Figure 1). wGRS1 and wGRS2 positively correlated with BMI loss (R = 0.50, P < 0.001 and R = 0.51, P < 0.001, for MHP and LF diets, respectively). In contrast, age and baseline energy intake negatively correlated with BMI loss (R = -0.26, P < 0.05 and R = -0.27, P < 0.01, respectively).

**Table 5. Multiple linear regression models using genetic, phenotypical, and environmental information to explain BMI loss as dependent variable in the MHP diet.**

| | | | SNPs | | | uGRS | wGRS |
|---|---|---|---|---|---|---|---|
| | LARS | | BSRP | | BSM | L/B/B | L/B/B |
| Variables | | adj. R² | AIC / AICC | BIC | | | |
| Age (years) | -0.105 | -0.105 | -0.109 | -0.112 | -0.112 | 0.098 | -0.106 |
| Sex | | | | | | | - |
| Baseline METs | | | | | | | - |
| Baseline energy intake | | | | | | | - |
| (1000 kcal) | | | | | | | |
| rs1801133 (*MTHFR*) | 2.344 | 2.344 | 2.523 | 2.724 | 2.724 | | |
| rs2605100 (*LYPLAL1*) | 2.147 | 2.147 | 2.149 | 2.149 | 2.149 | | |
| rs3123554 (*CNR2*) | 1.940 | 1.940 | 1.891 | 1.929 | 1.929 | | |
| rs1175544 (*PPARG*) | 0.813 | 0.813 | | | | | |
| rs10767664 (*BDNF*) | 1.719 | 1.719 | 1.752 | 1.920 | 1.920 | | |
| rs659366 (*UCP2*) | 1.240 | 1.240 | 1.252 | | | | |
| rs1052700 (*PLIN1*) | 2.837 | 2.837 | 2.871 | 2.955 | 2.955 | | |
| uGRS1 | | | | | | 1.682 | |
| wGRS1 | | | | | | | 0.994 |
| Constant | - | - | | - | - | - | - |
| | 13.327 | 13.327 | 12.890 | 12.492 | 12.492 | 12.443 | 12.805 |
| R² | 0.327 | 0.327 | 0.319 | 0.298 | 0.298 | 0.304 | 0.317 |
| Adj. R² | 0.266 | 0.266 | 0.265 | 0.250 | 0.250 | 0.289 | 0.302 |
| Optimism correction for | 0.111 | 0.111 | 0.096 | 0.086 | 0.086 | 0.020 | 0.022 |
| R² | | | | | | | |
| Optimism correction for | 0.122 | 0.122 | 0.103 | 0.092 | 0.092 | 0.021 | 0.023 |
| adj. R² | | | | | | | |
| Optimism-corrected R² | 0.216 | 0.216 | 0.223 | 0.212 | 0.212 | 0.284 | 0.295 |
| Optimism-corrected adj. | 0.144 | 0.144 | 0.162 | 0.158 | 0.158 | 0.268 | **0.279** |
| R² | | | | | | | |

| | | | | | | | |
|---|---|---|---|---|---|---|---|
| Data are expressed as β values. METSs: metabolic equivalents; uGRS1: unweighted genetic risk score 1; wGRS1: weighted genetic risk score 1; LARS: least-angle regression; BSRP: best subset regression procedure; BSM: bootstrapping stepwise method; AIC: akaike information criterion; AICC: corrected akaike information criterion; BIC: bayesian information criterion; MHP: moderately high-protein diet; L/B/B: LARS/BSRP/BSM. | | | | | | | |

**Table 6. Multiple linear regression models using genetic, phenotypical, and environmental information to explain BMI loss as dependent variable in the LF diet.**

| | | | SNPs | | | uGRS | wGRS |
|---|---|---|---|---|---|---|---|
| | LARS | | BSRP | | BSM | L/B/B | L/B/B |
| Variables | | adj. R² | AIC / AICC | BIC | | | |
| Age (years) | - | - | - | - | - | - | - |
| Sex | 0.499 | - | - | - | - | - | - |
| Baseline METs | - | - | - | - | - | - | - |
| Baseline energy intake | 0.836 | 0.892 | 0.937 | 0.958 | 0.937 | -0.884 | -0.858 |
| (1000 kcal) | | | | | | | |
| rs2943641 (*IRS1*) | 1.185 | 2.148 | - | - | - | | |
| rs10182181 (*ADCY3*) | 1.791 | 1.811 | 2.041 | 2.108 | 2.041 | | |
| rs1042713 (*ADRB2*) | 0.849 | 0.848 | - | - | - | | |
| rs1800544 (*ADRA2A*) | 1.830 | 1.806 | 1.882 | 2.089 | 1.882 | | |
| rs662799 (*APOA5*) | 1.582 | 1.509 | 1.909 | - | 1.909 | | |
| Rs6123837 (*GNAS*) | 1.377 | 1.369 | 1.562 | - | 1.562 | | |
| Rs3813929 (*HTR2C*) | 1.943 | 2.137 | 2.133 | 2.184 | 2.133 | | |
| uGRS2 | | | | | | 1.211 | |
| wGRS2 | | | | | | | 0.966 |
| Constant | - | - | -10.353 | - | - | -10.512 | - |
| | 11.291 | 10.770 | | 9.259 | 10.353 | | 10.882 |
| R² | 0.322 | 0.319 | 0.297 | 0.234 | 0.297 | 0.296 | 0.316 |
| Adj. R² | 0.257 | 0.262 | 0.253 | 0.203 | 0.253 | 0.282 | 0.303 |
| Optimism correction for R² | 0.099 | 0.087 | 0.063 | 0.040 | 0.063 | 0.017 | 0.016 |
| Optimism correction | 0.109 | 0.094 | 0.067 | 0.042 | 0.067 | 0.017 | 0.016 |
| for adj. R² | | | | | | | |
| Optimism-corrected R² | 0.223 | 0.232 | 0.234 | 0.194 | 0.234 | 0.279 | 0.300 |
| Optimism-corrected | 0.148 | 0.168 | 0.186 | 0.161 | 0.186 | 0.265 | **0.287** |
| adj. R² | | | | | | | |

| | | | | | | | |
|---|---|---|---|---|---|---|---|
| Data are expressed as β values. METSs: metabolic equivalents; uGRS2: unweighted genetic risk score 2; wGRS2: weighted genetic risk score 2; LARS: least-angle regression; BSRP: best subset regression procedure; BSM: bootstrapping stepwise method; AIC: akaike information criterion; AICC: corrected akaike information criterion; BIC: bayesian information criterion; LF: low-fat diet; L/B/B: LARS/BSRP/BSM. | | | | | | | |

Together, these findings showed that the genetic influence in weight reduction is dependent on interactions with diet composition.

According to these results, predictive values for BMI loss variance were found when analyzing individually the effect of each of the previously mentioned SNPs in the multiple linear regression models (see in tables 5 and 6 the results for each SNP.) In order to improve this outcome, unweigthed GRS were fitted with good results (Optimism-corrected adj. R² for uGRS for the MHP diet was 0.268, whereas the Optimism-corrected adj. R² for uGRS for the LF diet was 0.265). Moreover, two weigthed GRS (wGRS) were fitted, one for each diet, through combining the effect sizes of all risk genotypes (lower BMI losses) showing a Optimism-corrected adj. R² for wGRS for the MHP diet was 0.279, whereas the Optimism-corrected adj. R² for wGRS for the LF diet was 0.287. Thus, wGRS1 and wGRS2 were major predictors of BMI loss in the MHP and LF diets, respectively.

Related to other variants studied, surprisingly, higher energy intake at baseline was associated with higher BMI loss in the LF diet, but not in the MHP regimen. Based on these results, energy restriction should be based on the current calorie consumption rather than calculations of individual requirements or weight status when prescribing a LF diet. Surprisingly, the higher BMI loss was found in older subjects only in the MHP diet.

Furthermore, a decision algorithm for precision BMI loss (%) management using genetic, phenotypical, and environmental characteristics was depicted (Figure 2).Figure 2 shows the flowchart of the method and the results obtained for three patients using the method of the invention. At patient's visit, conventional data such as age and energy intake at baseline are obtained through clinical and nutritional evaluations. After blood sampling, genotyping of relevant SNPs influencing BMI loss was performed and wGRS were computed. Once genetic, phenotypical, and environmental information was registered, BMI loss was predicted by applying the corresponding model for MHP and LF diets (Z test, as previously explained in the material and method section). Subsequently, average BMI loss predictions by diet were compared using appropriate statistical tests. Thus, nutritional advice was given based on the prescription of a specific diet (if statistically significant differences between predictive values for MHP and LF diets) or taking into account individual food preferences (if no statistically significant differences between predictive values for MHP and LF diets), as shown in Figure 2, wherein the individual could choose between the MHP or the LF diet.

### Citation List

### Non Patent Literature

Abete I, Astrup A, Martinez JA, Thorsdottir I, Zulet MA. Obesity and the metabolic syndrome: role of different dietary macronutrient distribution patterns and specific nutritional components on weight loss and maintenance. Nutr Rev. 2010;68(4):214-31.
Martinez JA, Navas-Carretero S, Saris WH, Astrup A. Personalized weight loss strategies-the role of macronutrient distribution. Nat Rev Endocrinol. 2014;10(12):749-60.
Bray GA, Ryan DH, Johnson W, Champagne CM, Johnson CM, Rood J, Williamson DA, Sacks FM. Markers of dietary protein intake are associated with successful weight loss in the POUNDS Lost trial. Clin Obes. 2017;7(3): 166-175.
Qi L. Gene-diet interaction and weight loss. Curr Opin Lipidol. 2014;25(1):27-34.
Goni L, Cuervo M, Milagro FI, Martinez JA. Future Perspectives of Personalized Weight Loss Interventions Based on Nutrigenetic, Epigenetic, and Metagenomic Data. J Nutr. 2016;146(4):905S-912S.
Sandholt CH, Allin KH, Toft U, Borglykke A, Ribel-Madsen R, Sparso T, Justesen JM, Harder MN, Jørgensen T, Hansen T, Pedersen O. The effect of GWAS identified BMI loci on changes in body weight among middle-aged Danes during a five-year period. Obesity (Silver Spring). 2014;22(3):901-8.
Cha S, Kang JH, Lee JH, Kim J, Kim H, Yang YJ, Park WY, Kim J. Impact of Genetic Variants on the Individual Potential for Body Fat Loss. Nutrients. 2018;10(3). pii: E266. doi: 10.3390/nu10030266.
Peter I, McCaffery JM, Kelley-Hedgepeth A, Hakonarson H, Reis S, Wagenknecht LE, Kopin AS, Huggins GS; Genetics Subgroup of the Look AHEAD Study. Association of type 2 diabetes susceptibility loci with one-year weight loss in the look AHEAD clinical trial. Obesity (Silver Spring). 2012;20(8):1675-82.
Lopez-Legarrea P, de la Iglesia R, Abete I, Navas-Carretero S, Martinez JA, Zulet MA. The protein type within a hypocaloric diet affects obesity-related inflammation: the RESMENA project. Nutrition. 2014 Apr;30(4):424-9. doi: 10.1016/j.nut.2013.09.009.
Efron B, Hastie T, Johnstone I, Tibshirani R. Least angle regression. Ann Statist. 2004;32(2):407-499.
Lindsey C, Sheather S. Variable selection in linear regression. Stata Journal. 2010;10(4): 650-669.
Austin PC, Tu JV. Bootstrap Methods for Developing Predictive Models. Am Stat. 2004; 58(2):131-137.
Lopez-Legarrea P, de la Iglesia R, Abete I, Bondia-Pons I, Navas-Carretero S, Forga L, Martinez JA, Zulet MA. Short-term role of the dietary total antioxidant capacity in two hypocaloric regimes on obese with metabolic syndrome symptoms: the RESMENA randomized controlled trial. Nutr Metab (Lond). 2013;10(1):22.
de la Iglesia R, Lopez-Legarrea P, Abete I, Bondia-Pons I, Navas-Carretero S, Forga L, Martinez JA, Zulet MA. A new dietary strategy for long-term treatment of the metabolic syndrome is compared with the American Heart Association (AHA) guidelines: the MEtabolic Syndrome REduction in NAvarra (RESMENA) project. Br J Nutr.2014;111(4):643-52.
Whitworth JA, Chalmers J. World health organisation-international society of hypertension (WHO/ISH) hypertension guidelines. Clin Exp Hypertens. 2004;26(7-8):747-52.
Tremblay AJ, Morrissette H, Gagné JM, Bergeron J, Gagné C, Couture P. Validation of the Friedewald formula for the determination of low-density lipoprotein cholesterol compared with beta-quantification in a large population. Clin Biochem. 2004;37(9):785-90.
Navarro-González D, Sánchez-Íñigo L, Pastrana-Delgado J, Fernandez-Montero A, Martinez JA. Triglyceride-glucose index (TyG index) in comparison with fasting plasma glucose improved diabetes prediction in patients with normal fasting glucose: The Vascular-Metabolic CUN cohort. Prev Med. 2016; 86:99-105.
Martin-Moreno JM, Boyle P, Gorgojo L, Maisonneuve P, Fernandez-Rodriguez JC, Salvini S, Willett WC. Development and validation of a food frequency questionnaire in Spain. Int J Epidemiol. 1993;22(3):512-9.
de la Fuente-Arrillaga C, Ruiz ZV, Bes-Rastrollo M, Sampson L, Martinez-Gonzalez MA. Reproducibility of an FFQ validated in Spain. Public Health Nutr. 2010;13(9):1364-72.
Moreiras O, Carbajal A, Cabrera L, Cuadrado C. Tablas de Composition de Alimentos, 16th ed; Piramide: Madrid, Spain, 2013.
Martínez-González MA, López-Fontana C, Varo JJ, Sanchez-Villegas A, Martinez JA. Validation of the Spanish version of the physical activity questionnaire used in the Nurses' Health Study and the Health Professionals' Follow-up Study. Public Health Nutr. 2005;8(7):920-7.
Basterra-Gortari FJ, Bes-Rastrollo M, Pardo-Fernández M, Forga L, Martinez JA, Martínez-González MA. Changes in weight and physical activity over two years in Spanish alumni. Med Sci Sports Exerc.
2009;41(3):516-22.
Ramos-Lopez O, Riezu-Boj JI, Milagro FI, Goni L, Cuervo M, Martinez JA. Differential lipid metabolism outcomes associated with ADRB2 gene polymorphisms in response to two dietary interventions in overweight/obese subjects. Nutr Metab Cardiovasc Dis. 2018a;28(2): 165-172.
Handjieva-Darlenska T, Holst C, Grau K, Blaak E, Martinez JA, Oppert JM, Taylor MA, Sørensen TI, Astrup A. Clinical correlates of weight loss and attrition during a 10-week dietary intervention study: results from the NUGENOB project. Obes Facts. 2012;5(6):928-36.
Ramos-Lopez O, Riezu-Boj JI, Milagro FI, Goni L, Cuervo M, Martinez JA. Association of the Gly482Ser PPARGC1A gene variant with different cholesterol outcomes in response to two energy-restricted diets in subjects with excessive weight. Nutrition. 2018b;47:83-89.
Guo F, Zhou Y, Song H, Zhao J, Shen H, Zhao B, Liu F, Jiang X. Next generation sequencing of SNPs using the HID-lon AmpliSeq™ Identity Panel on the Ion Torrent PGM™ platform. Forensic Sci Int Genet. 2016;25:73-84.
Hung CF, Breen G, Czamara D, Corre T, Wolf C, Kloiber S, Bergmann S, Craddock N, Gill M, Holsboer F, Jones L, Jones I, Korszun A, Kutalik Z, Lucae S, Maier W, Mors O, Owen MJ, Rice J, Rietschel M, Uher R, Vollenweider P, Waeber G, Craig IW, Farmer AE, Lewis CM, Müller-Myhsok B, Preisig M, McGuffin P, Rivera M. A genetic risk score combining 32 SNPs is associated with body mass index and improves obesity prediction in people with major depressive disorder. BMC Med. 2015;13:86.
Ou Z, Tempelman RJ, Steibel JP, Ernst CW, Bates RO, Bello NM. Genomic Prediction Accounting for Residual Heteroskedasticity. G3 (Bethesda). 2015;6(1):1-13.
Iacobucci D, Schneider MJ, Popovich DL, Bakamitsos GA. Mean centering, multicollinearity, and moderators in multiple regression: The reconciliation redux. Behav Res Methods. 2017;49(1):403-404.
Harrell FE Jr, Lee KL, Mark DB. Multivariable prognostic models: issues in developing models, evaluating assumptions and adequacy, and measuring and reducing errors. Stat Med. 1996;15(4):361-87.

## Claims

1. An *in vitro* method to predict the percentage of body mass index (BMI) reduction in response to a diet in a patient with a BMI of at least 25 kg/m² comprising detecting in an isolated sample of the subject the genotype of one or more single nucleotide polymorphisms (SNPs) selected from the following list: rs1801133 (*MTHFR*), rs1052700 (*PLIN1*), rs2605100 (*LYPLAL1*), rs3123554 (*CNR2*), rs10767664 (*BDNF*), rs659366 (*UCP2*) and combinations thereof; and the detection of the genotype of one or more SNPs selected from the following list: rs1800544 (*ADRA2A*), rs3813929 (*HTR2C*), rs10182181 (*ADCY3*), rs2943641 (*IRS1*), rs6123837 (*GNAS*), rs662799 (*APOA5*), rs1042713 (*ADRB2*) and combinations thereof; wherein the diet is an energy-restricted diet selected from a low-fat (LF) diet wherein the total energy (E) intake from lipids is 20-25%; or, alternatively, the energy-restricted diet is a moderately high-protein (MHP) diet wherein the E from proteins is 25-35%.

2. An *in vitro* method to predict the percentage of BMI reduction in response to a diet according to claim 1, wherein the diet is an energy-restricted diet selected from a low-fat (LF) diet wherein the total energy (E) intake from carbohydrates is 55-65%, E from proteins is 15-20% and E from lipids is 20-25%; or, alternatively, the energy-restricted diet is a moderately high-protein (MHP) diet wherein the E from carbohydrates is 40-50%, E from proteins is 25-35% and E from lipids is 28-35%.

3. The *in vitro* method to predict the percentage of BMI reduction in response to a diet according to claims 1or2 comprising detecting the genotype of the SNPs rs1801133, rs1052700, rs2605100, rs3123554, rs10767664 and rs659366; and the detection of the genotype of the SNPs rs1800544, rs3813929, rs10182181, rs2943641, rs6123837, rs662799 and rs1042713.

4. The *in vitro* method to predict the percentage of BMI reduction in response to a diet according to any one of claims 1 to 3 wherein, when one or more of the genotypes selected from the following list is detected, it is indicative of reduction of the percentage of BMI of the MHP diet as defined in any one of claims 1 to 3: AA of rs1801133, TT of rs1052700, GG of rs2605100, AA or AG of rs3123554, TA of rs10767664, CC of rs659366 and combinations thereof; or alternatively, when one or more of the genotypes selected from the following list is detected, it is indicative of reduction of percentage of BMI of the LF diet as defined in any one of claims 1 to 3: GG or GC of rs1800544, CC or TT of rs3813929, AG or GG of rs10182181, TC or CC of rs2943641, GA of rs6123837, GG or AA of rs662799, GA of rs1042713 and combinations thereof.

5. The *in vitro* method to predict the percentage of BMI reduction in response to a diet according to claim 4 wherein, when the following genotypes are detected it is indicative of reduction of the percentage of BMI of the MHP diet as defined in any one of claims 1 to 4: AA of rs1801133, TT of rs1052700, GG of rs2605100, AA or AG of rs3123554, TA of rs10767664 and CC of rs659366; and/or, when the following genotypes are detected, it is indicative of reduction of the percentage of BMI of the LF diet as defined in any one of claims 1 to 4: GG or GC of rs1800544, CC or TT of rs3813929, AG or GG of rs10182181, TC or CC of rs2943641, GA of rs6123837, GG or AA of rs662799 and GA of rs1042713.

6. An *in vitro* method for deciding or recommending a patient with a BMI of at least 25 kg/m² to follow an energy-restricted diet for reduction of the percentage of BMI, selected from a LF diet wherein the total E intake from lipids is 20-25%; or a MHP diet wherein the E from proteins is 25-35%; wherein the method comprises detecting in an isolated sample of the subject the genotype of one or more SNPs selected from the following list: rs1801133 (*MTHFR*), rs1052700 (*PLIN1*), rs2605100 (*LYPLAL1*), rs3123554 (*CNR2*), rs10767664 (*BDNF*), rs659366 (*UCP2*) and combinations thereof; and the detection of the genotype of at least one or more SNPs selected form the following list: rs1800544 (*ADRA2A*), rs3813929 (*HTR2C*), rs10182181 (*ADCY3*), rs2943641 (*IRS1*), rs6123837 (*GNAS*), rs662799 (*APOA5*), rs1042713 (*ADRB2*) and combinations thereof.

7. An *in vitro* method for deciding or recommending a patient with a BMI of at least 25 kg/m² to follow an energy-restricted diet according to claim 7, wherein in the LF diet the total E intake from carbohydrates is 55-65%, E from proteins is 15-20% and E from lipids is 20-25%; or wherein in the MHP diet the E from carbohydrates is 40-50%, E from proteins is 25-35% and E from lipids is 28-35%.

8. The *in vitro* method for deciding or recommending a patient with a BMI of at least 25 kg/m² to follow an energy-restricted diet according to any one of claims 6 or 7 wherein the method comprises detecting the genotype of the SNPs of the following list: rs1801133 (*MTHFR*), rs1052700 (*PLIN1*), rs2605100 (*LYPLAL1*), rs3123554 (*CNR2*), rs10767664 (*BDNF*) and rs659366 (*UCP2*); and the detection of the SNPs of the following list: rs1800544 (*ADRA2A*), rs3813929 (*HTR2C*), rs10182181 (*ADCY3*), rs2943641 (*IRS1*), rs6123837 (*GNAS*), rs662799 (*APOA5*) and rs1042713 (*ADRB2*).

9. The *in vitro* method for deciding or recommending a patient with BMI of at least 25 kg/m² to follow an energy-restricted diet according to any one of claims 6 to 8 wherein the method comprises detecting one or more of the genotypes selected from the following list: AA of rs1801133, TT of rs1052700, GG of rs2605100, AA or AG of rs3123554, TA of rs10767664, CC of rs659366 and combinations thereof; and/or detecting one or more of the genotypes selected from the following list: GG or GC of rs1800544, CC or TT of rs3813929, AG or GG of rs10182181, TC or CC of rs2943641, GA of rs6123837, GG or AA of rs662799, GA of rs1042713 and combinations thereof.

10. The *in vitro* method for deciding or recommending a patient with BMI of at least 25 kg/m² to follow an energy-restricted diet according to any one of claim 9 wherein the method comprises detecting the genotypes selected from the following list: AA of rs1801133, TT of rs1052700, GG of rs2605100, AA or AG of rs3123554, TA of rs10767664 and CC of rs659366; and detecting the genotypes selected from the following list 5: GG or GC of rs1800544, CC or TT of rs3813929, AG or GG of rs10182181, TC or CC of rs2943641, GA of rs6123837, GG or AA of rs662799 and GA of rs1042713.

11. The *in vitro* method to predict the percentage of body mass index (BMI) reduction in response to a diet of any one of claims 1 to 5 or the *in vitro* method for deciding or recommending a patient with a BMI of at least 25 kg/m² to follow an energy-restricted diet according to any one of claims 6 to 10, further comprising determining a genetic risk score of the percentage of BMI reduction.

12. The *in vitro* method for deciding or recommending a patient with BMI of at least 25 kg/m² to follow an energy-restricted diet according to any one of claims 6 to 11 further comprising considering one or more of the clinical parameters and/or features of the subject selected form age, baseline energy intake, gender, weight, BMI, total body fat, visceral fat, waist circumference, blood pressure, systolic blood pressure, diastolic blood reassure, triglyceride level, hypercholesterolemia, total cholesterol levels high-density lipoprotein cholesterol level, low-density lipoprotein cholesterol triglycerides level, triglyceride-glucose index, energy dietary intake per day, physical activity, diabetes status, hypertension and combinations thereof.

13. The *in vitro* method for deciding or recommending a patient with BMI of at least 25 kg/m² to follow an energy-restricted diet according to claim 12 considering age for deciding or recommending the MHP diet as defined in any one of claims 6 to 12, and considering baseline energy intake for deciding or recommending the LF diet as defined in any one of claims 6 to 12.

14. The *in vitro* method for deciding or recommending a patient with a BMI of at least 25 kg/m² to follow an energy-restricted diet according to claim 13, further comprising obtaining a predictive value derived from the genetic risk score and the age in the case of the MHP diet; and obtaining a predictive value derived from the genetic risk score and the baseline energy intake for the LF diet; and comparing statistically the predictive value of the percentage of BMI reduction between the diet MHP and the LF diet.

15. The *in vitro* method for deciding or recommending a patient with BMI of at least 25 kg/m² to follow an energy-restricted diet according to claim 14, wherein if after the statistical comparison the subject is considered at risk of non-reduction of percentage of BMI using diet MHP as defined in any one of claims 6 to 14, then the LF diet as defined in any one of claims 6 to 14 is prescribed; whereas if the subject is considered at risk of non-reduction of percentage of BMI using diet LF as defined in any one of claims 6 to14, then the MHP diet as defined in any one of claims 6 to 14 is prescribed; whereas when the predictive value is similar to both diets, then any of them can be prescribed.
